(19) Europäisches Patentamt
European Patent Office
Office européen des brevets

(11) **EP 2 350 036 B1**

(12) **FASCICULE DE BREVET EUROPEEN**

(45) Date de publication et mention de la délivrance du brevet:
**15.04.2015 Bulletin 2015/16**

(21) Numéro de dépôt: **09815693.8**

(22) Date de dépôt: **25.09.2009**

(51) Int Cl.:
***C07D 307/58*** *(2006.01)* ***C07D 409/04*** *(2006.01)*
***C07D 409/06*** *(2006.01)* ***A61K 8/49*** *(2006.01)*
***A61Q 17/04*** *(2006.01)*

(86) Numéro de dépôt international:
**PCT/EP2009/062476**

(87) Numéro de publication internationale:
**WO 2010/034827 (01.04.2010 Gazette 2010/13)**

(54) **DERIVES DE METHYLENE FURANONE ET UTILISATION DE CES DERIVES EN TANT QU'AGENT PHOTOPROTECTEUR OU ANTIOXYDANT OU DEPIGMENTANT DANS DES COMPOSITIONS A USAGE COSMETIQUE OU DERMATOLOGIQUE.**

METHYLEN-FURANON-DERIVATE UND VERWENDUNG DIESER DERIVATE ALS LICHTSCHUTZ- ODER ANTIOXIDATIONS- ODER DEPIGMENTIERUNGSMITTEL IN KOSMETISCHEN ODER DERMATOLOGISCHEN ZUSAMMENSETZUNGEN

METHYLENE FURANONE DERIVATIVES AND USE OF SAID DERIVATIVES AS A PHOTOPROTECTING OR ANTIOXIDANT OR DEPIGMENTATION AGENT IN COSMETIC OR DERMATOLOGICAL COMPOSITIONS

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **25.09.2008 FR 0856441**

(43) Date de publication de la demande:
**03.08.2011 Bulletin 2011/31**

(73) Titulaire: **Pierre Fabre Dermo-Cosmétique**
**92100 Boulogne-Billancourt (FR)**

(72) Inventeurs:
- **MARCHAL, Estelle**
  **F-54910 Valleroy (FR)**
- **URIAC, Philippe**
  **F-35740 Pace (FR)**
- **BRUNEL, Yves**
  **F-81150 Marssac-sur-tarn (FR)**
- **POIGNY, Stéphane**
  **F-31600 Saubens (FR)**

(74) Mandataire: **Regimbeau**
**20, rue de Chazelles**
**75847 Paris Cedex 17 (FR)**

(56) Documents cités:
**WO-A-2008/040097 US-B1- 6 258 963**

- **ANTONIOLETTI R ET AL: "A NEW SYNTHESIS OF 5 METHYLENE-2-5H-FURANONE DERIVATIVES" TETRAHEDRON, vol. 40, no. 19, 1984, pages 3805-3808, XP002523883 ISSN: 0040-4020**
- **ROSSI R ET AL: "Palladium-catalyzed synthesis of stereodefined 3-[(1,1-unsymmetrically disubstituted)methylidene]isobenzofuran-1 ( 3H)-ones and stereodefined 5-[(1,1-unsymmetrically disubstituted)methylidene] furan-2(5H)-ones " TETRAHEDRON LETTERS, vol. 41, no. 27, 1 juillet 2000 (2000-07-01), pages 5281-5286, XP004222055 ISSN: 0040-4039**
- **KAR A ET AL: "A facile synthesis of rubrolide E<1>" SYNTHESIS 20050902 DE, no. 14, 2 septembre 2005 (2005-09-02), pages 2284-2286, XP002523884 ISSN: 0039-7881**
- **SAALFRANK R W ET AL: "CONVENIENT SYNTHESIS OF 5 ALKYLIDENE-2-5H-FURANONES 2-5H FURANONES AND 2 ETHOXYFURANS" TETRAHEDRON, vol. 44, no. 16, 1988, pages 5095-5100, XP002523885 ISSN: 0040-4020**

EP 2 350 036 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- PRIM D. ET AL: "Synthesis and stereochemistry of beta-aryl-beta-haloacroleins: useful intermediates for the preparation of (Z) and (E)-2-en-4-ynecarbaldehydes and for the synthesis of rubrolides" JOURNAL OF THE CHEMICAL SOCIETY, PERKIN TRANSACTIONS 2, 1999, pages 1175-1180, XP002523886 ISSN: 1472-779X
- BOUKOUVALAS J ET AL: "Facile Access to 4-Aryl-2(5H)-furanones by Suzuki Cross Coupling: Efficient Synthesis of Rubrolides C and E" TETRAHEDRON LETTERS, vol. 39, no. 42, 15 octobre 1998 (1998-10-15), pages 7665-7668, XP004134276 ISSN: 0040-4039
- WU J ET AL: "Palladium-catalyzed cross-coupling reactions of 4-tosyl-2(5H)-furanone with boronic acids: A facile and efficient route to generate 4-substituted 2(5H)-furanones" JOURNAL OF ORGANIC CHEMISTRY, vol. 68, no. 2, 24 janvier 2003 (2003-01-24), pages 670-673, XP002523887 ISSN: 0022-3263
- DATABASE CA [Online] CHEMICAL ABSTRACTS SERVICE, COLUMBUS, OHIO, US; 1983, CHELLAR, N. S. ET AL: "Condensation of p-substituted 3-aryl-2-butenolides with aromatic and heterocyclic aldehydes" XP002523888 extrait de STN Database accession no. 1986: 109384 & KHIMIYA I KHIM. TEKHNOL., EREVAN , (2), 97-101 FROM: REF. ZH., KHIM. 1985, ABSTR. NO. 9ZH189, 1983,
- DATABASE CA [Online] CHEMICAL ABSTRACTS SERVICE, COLUMBUS, OHIO, US; 1980, SIDKY, M. M. ET AL: "Organophosphorus compounds, XXX. The reaction of fluorenylidenetriphenylphosphorane with maleic, phthalic and thiophthalic anhydrides" XP002550711 extrait de STN Database accession no. 1980:639115 & SIDKY, M. M. ET AL.: "Organophosphorus compounds, XXX. The reaction of fluorenylidenetriphenylphosphorane with maleic, phthalic and thiophthalic anhydrides" PHOSPHORUS AND SULFUR AND THE RELATED ELEMENTS, vol. 8, no. 3, 1980, pages 365-367, ISSN: 0308-664X
- ERGIN VOLKAN ET AL: "Carbonyl Stress in Aging Process: Role of Vitamins and Phytochemicals as Redox Regulators", AGING AND DISEASE, vol. 4, no. 5, October 2013 (2013-10), pages 276-294, ISSN: 2152-5250(print)
- GANDHI SONIA ET AL: "Mechanism of Oxidative Stress in Neurodegeneration", OXIDATIVE MEDICINE AND CELLULAR LONGEVITY, 2012, page Article No.: 428010, ISSN: 1942-0900(print)

## Description

La présente invention concerne des dérivés de méthylène furanone, leur synthèse, ainsi que leur utilisation en tant qu'agent photoprotecteur ou antioxydant ou dépigmentant dans des compositions à usage cosmétique ou dermatologique.

Les dérivés de méthylène furanone sont des molécules très abondantes dans la nature. On les retrouve par exemple dans les constituants de l'odeur de céleri sous forme d'alkylidène-benzofuranone (**a**). Ils sont également représentés dans les lichens par l'acide pulvinique (**b**) responsable de leur pigmentation jaune. Ils sont également connus pour présenter une grande variété d'activités biologiques. Ainsi, la protoanémonine (**c**), possède des propriétés antimicrobiennes et antifongiques, le rubrolide A (**d**) des propriétés antibactériennes et la dihydroxélurine (**e**) inhibe la synthèse du cholestérol.

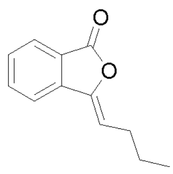

alkylidènephtalide **a**

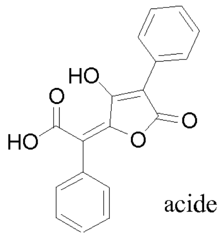

acide pulvinique **b**

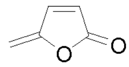

protoanémonine **c**

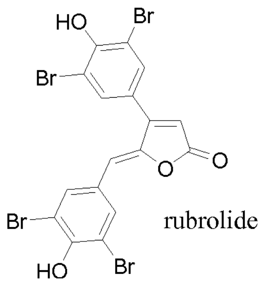

rubrolide A **d**

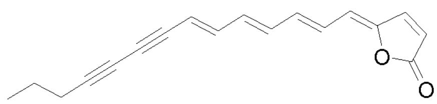

dihydroxérulin **e**

US 6,258,963 B1 décrit des dérivés de benzylidène-gamma-butyrolactones comme absorbeur UV pour utilisation pharmaceutique ou cosmétique.

La présente invention concerne les dérivés de méthylène furanone de formule générale (I)

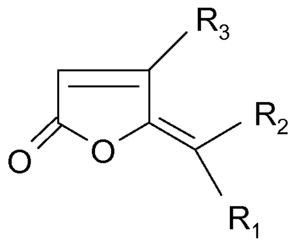

(I)

sous leurs formes isomères (E) ou (Z), pures ou en mélange,
dans laquelle $R_3$ représente un hydrogène et où :

- $R_1$ et $R_2$ sont identiques et représentent
  - un radical phényle substitué par un ou plusieurs radicaux
    - ◦ alkyl($C_{1-5}$), alkyl($C_{1-4}$)oxy,
    - ◦ hydroxy,
    - ◦ méthylthio,
    - ◦ halogéno, ou
    - ◦ un groupement amino éventuellement substitué par un radical alkyl($C_{1-4}$), alkyl($C_{1-4}$)oxycarbonyle,

- un radical thiophényle,
- un radical thionaphtènyle,
- un radical thiazolyle,
- un radical pyridinyle,
- un radical benzomorpholinyle,
- un radical naphtyle

et
à la condition toutefois que $R_1$ et $R_2$ ne représentent pas simultanément un radical paraméthoxyphényle.

L'invention concerne également les dérivés de méthylène furanone de formule générale (I), sous leurs formes isomères (E) ou (Z), pures ou en mélange, dans laquelle :

- R3 représente un hydrogène
- R1 et R2 sont identiques et représentent
  - un radical phényle substitué par un ou plusieurs radicaux
    - alkyl($C_{1-4}$)oxy,
    - hydroxy,
  - un radical thiophényle,

et à la condition toutefois que $R_1$ et $R_2$ ne représentent pas simultanément un radical paraméthoxyphényle.

L'invention s'étend également aux dérivés de méthylène furanone de formule générale (I), sous leurs formes isomères (E) ou (Z), pures ou en mélange, dans laquelle :

- R3 représente un hydrogène
- R1 et R2 sont identiques et représentent
  - un radical phényle substitué par un ou plusieurs radicaux
    - hydroxy

Plus particulièrement, l'invention concerne les composés suivants, dérivés de méthylène furanone de formule générale (I), sous leurs formes isomères (E) ou (Z), pures ou en mélange, , choisis parmi :

- 5-(bis(3,4-diméthoxyphényl)méthylène)furane-2(*5H*)-one ;
- 5-(dithiophène-3-ylméthylène)furane-2(*5H*)-one ;
- 5-bis(4-hydroxyphénylcarbamate de *tert*-butyl)furane-2(*5H*)-one ;
- 5-(bis(2-fluorophényl)méthylène)furane-2(*5H*)-one ;
- 5-(bis(4-(méthylthio)phényl)méthylène)furan-2(5H)-one
- 5-(bis(4-(diméthylamino)phényl)méthylène)furan-2(5H)-one
- 5-(bis(4-méthyl-3,4-dihydro-2H-benzo[b][1,4]oxazin-7-yl)méthylène)furan-2(5H)-one
- 5-(dithiazol-2-ylméthylène)furan-2(5H)-one
- 5-(bis(4-fluorophényl)méthylène)furan-2(5H)-one
- 5-(bis(4-hydroxy-3,5-diméthylphényl)méthylène)furan-2(5H)-one
- 5-(bis(3,4,5-trihydroxyphényl)méthylène)furan-2(5H)-one
- 5-(bis(3,4-dihydroxyphényl)méthylène)furan-2(5H)-one
- 5-(bis(4-hydroxy-3-méthoxyphényl)méthylène)furan-2(5H)-one
- 5-(bis(4-hydroxy-3,5-diméthoxyphényl)méthylène)furan-2(5H)-one
- 5-(bis(4-hydroxyphényl)méthylène)furan-2(5H)-one
- 5-(bis(2,4-dimethoxyphenyl)methylene)furan-2(5H)-one
- 5-(bis(2,4-dihydroxyphenyl)methylene)furan-2(5H)-one

L'invention concerne encore des compositions dermocosmétiques comprenant un excipient cosmétiquement acceptable et un dérivé de méthylène furanone tel que défini ci-dessus en tant qu'agent photoprotecteur ou agent antioxydant.

L'invention concerne également des dérivés de formule générale (I) :

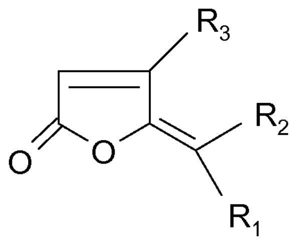

(I)

sous leurs formes isomères (E) ou (Z), pures ou en mélange, l'un des radicaux $R_1$ et $R_3$ représentant nécessairement un hydrogène, dans laquelle $R_1$, $R_2$, $R_3$ sont identiques ou différents et représentent :

- un radical phényle éventuellement substitué par un ou plusieurs radicaux
    - ◦ alkyl($C_{1-5}$), alkyl($C_{1-4}$)oxy,
    - ◦ hydroxy,
    - ◦ méthylthio,
    - ◦ halogéno, ou
    - ◦ un groupement amino éventuellement substitué par un radical alkyl($C_{1-4}$), alkyl($C_{1-4}$)oxycarbonyle,
- un radical thiophényle,
- un radical thionaphtènyle,
- un radical thiazolyle,
- un radical pyridinyle,
- un radical benzomorpholinyle,
- un radical naphtyle.
- R1 et R2 sont reliés entre eux et représentent un radical fluorenyl

Pour leur utilisation dermatologique en tant qu'agent photoprotecteur, ou agent antioxydant, ainsi que l'utilisation cosmétique des mêmes dérivés en tant qu'agent photoprotecteur, ou agent antioxydant

Dans une mise en oeuvre particulière de l'invention les dits dérivés de formule générale (I) sont sous leurs formes isomères (E) ou (Z), pures ou en mélange, tels que

- $R_3$ représente un hydrogène, et
- $R_1$ et $R_2$ sont identiques ou différents et représentent
    - un radical phényle éventuellement substitué par un ou plusieurs radicaux
        - ◦ alkyl($C_{1-5}$), alkyl($C_{1-4}$)oxy,
        - ◦ hydroxy,
        - ◦ méthylthio,
        - ◦ halogéno, ou
        - ◦ un groupement amino éventuellement substitué par un radical alkyl($C_{1-4}$), alkyl($C_{1-4}$)oxycarbonyle,
    - un radical thiophényle,
    - un radical thionaphtènyle,
    - un radical thiazolyle,
    - un radical pyridinyle,
    - un radical benzomorpholinyle,
    - un radical naphtyle.
    - R1 et R2 sont reliés entre eux et représentent un radical fluorenyl.

L'invention concerne également l'utilisation de dérivés de formule générale (I) sous leurs formes isomères (E) ou (Z), pures ou en mélange, tels que :

- R3 représente un hydrogène
- R1 et R2 sont identiques et représentent

- un radical phényle substitué par un ou plusieurs radicaux hydroxy en tant qu'agent dépigmentant.

L'invention concerne des compositions dermocosmétiques contenant un dérivé de méthylène furanone tel que défini ci-dessus en tant qu' agent dépigmentant en association avec un excipient cosmétiquement acceptable.

Les composés de formule générale (I) selon l'invention peuvent être préparés selon les schémas de synthèse ci-dessous.

1. Via le couplage de Suzuki- Miyaura

Afin d'obtenir les dérivés de méthylène furanone **4** et **5** la stratégie de synthèse retenue consiste à transformer l'acide 3,5-dibromolévulinique 1 (Manny, A. J. ; Kjelleberg, S. ; Kumar, N. ; de Nys, R. ; Read, R. W. ; Steinberg, P., Tetrahedron 1997, 53, 158813-15826.) en dibromofuranones **2** et **3.** Leur formation est dépendante des conditions employées : à 110°C dans l'acide sulfurique concentré **2** sera obtenue majoritairement, et **3** par traitement dans l'oléum à 65°C.

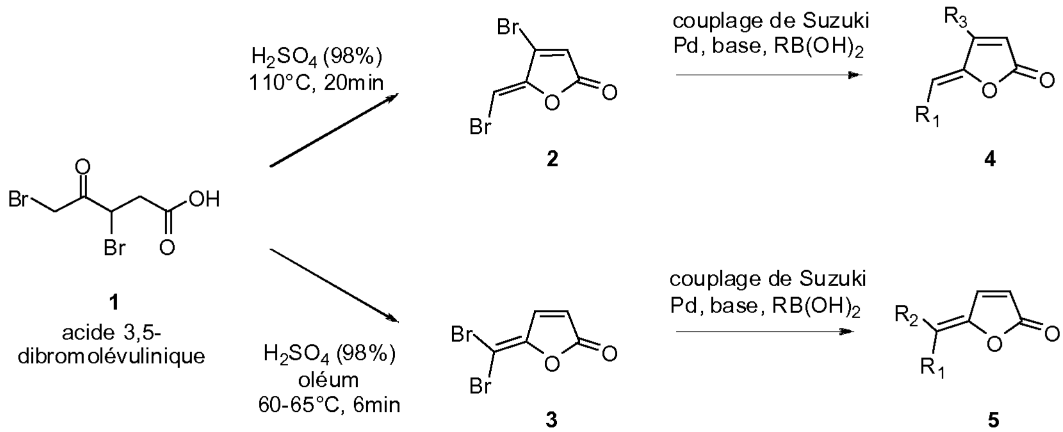


**1**
acide 3,5-dibromolévulinique

**2** **4** **3** **5**

Puis les atomes de brome sont substitués par des groupes aromatiques (de manière concomitante ou séquentielle) par un couplage de Suzuki-Miyaura.

Le couplage de Suzuki-Miyaura catalyse la formation de liaisons carbone-carbone entre un composé organoboré (porteur d'un carbone sp, $sp^2$ ou $sp^3$) et différents électrophiles, catalysés par des complexes au palladium, en présence d'une base.

Le choix des bases dépend de l'espèce organoborée et du partenaire électrophile utilisé. Ce sont principalement des bases minérales, telles que : $Na_2CO_3$, NaOH, $K_2CO_3$, $NaHCO_3$, $K_3PO_4$, NaOAc, CsF...

Les acides boroniques les plus couramment employés sont peu sensibles à l'air. De plus un très grand nombre est disponible commercialement. Par contre ces acides forment *in situ* des trimères cycliques (boroxines), et il faut en général les utiliser en excès.

Les esters boroniques peuvent être employés en quantité stoechiométrique. Les alkyles boranes sont également d'usage courant. Les catalyseurs les plus couramment utilisés dans le couplage de Suzuki-Miyaura sont : $Pd(PPh_3)_4$, $Pd(OAc)_2$, $Pd(dba)_2$, $Pd_2(dba)_3$, $PdCl_2$.

**Préparation des lactones dibromées 2 et 3**

La synthèse des bromolactones 2 et 3 est réalisée à partir de l'acide 3,5-dibromolévulinique **1,** lui même obtenu par bromation de l'acide lévulinique. En présence d'acide sulfurique concentré à chaud, la dibromométhylènefuranone **2** est obtenue majoritairement avec un rendement de 35 %. La préparation de dibromométhylènefuranone **3** nécessite la présence d'un mélange d'oléum et d'acide sulfurique concentré. Le rendement de cette réaction est seulement de 20 %. Ces faibles rendements peuvent être expliqués par les conditions drastiques utilisées et la stabilité moyenne de ces composés bromés. C'est à ce jour la seule méthode connue pour les synthétiser.

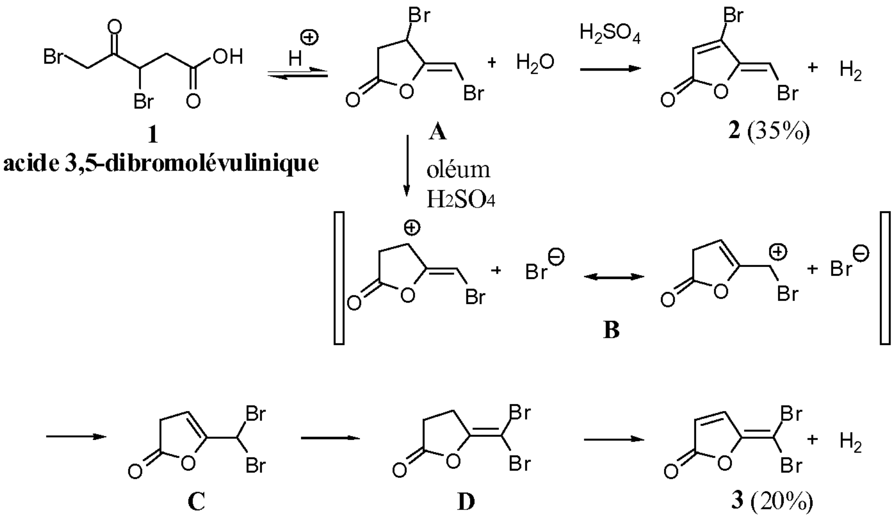


1
acide 3,5-dibromolévulinique
A
2 (35%)
B
C
D
3 (20%)

Le premier intermédiaire commun **A** de cette réaction est issu de la cyclisation de l'acide 3,5-dibromolévulinique. La voie acide sulfurique concentré peut alors donner le substrat **2** par déshydrogénation. Pour la seconde voie, la présence d'oléum permet la formation d'un carbocation allylique **B** et provoque ainsi la transposition du brome (intermédiaire **C**). Ensuite la migration de la double liaison donne le composé **D** qui, sous l'influence du caractère oxydant de l'acide sulfurique, conduit à la dibromométhylènefuranone **3.**

**Disubstitution des lactones bromées 2 et 3 par couplage de Suzuki-Miyaura**

La mise en oeuvre du couplage de Suzuki-Miyaura sur les furanones bromées **2** et **3** nous a permis de réaliser deux familles de dérivés de méthylène furanone, l'une disubstituée par des aromatiques identiques et l'autre disubstituée par des aromatiques différents.

<u>Cas des dérivés de méthylène furanone **4** et **5**, substitués par des aromatiques identiques.</u>

Pour réaliser ces couplages deux méthodes peuvent être appliquées :

- Méthode A
  $ArB(OH)_3$, 3 éq.
  $Pd(PPh_3)_4$, 3 mol %.
  $K_2CO_3$, 3 éq.
  toluène/$H_2O$/EtOH
  100°C, 4 h
- Méthode B
  $ArB(OH)_3$, 3 éq.
  $Pd(OAc)_2$ 1 mol %,
  S-Phos 2-10 2 mol %
  $K_3PO_4$, 3 éq.
  toluène, 100°C, 4 h

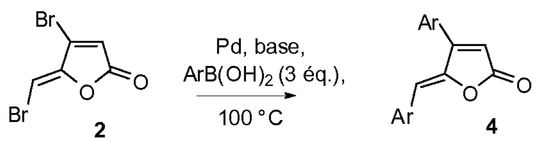


2
4

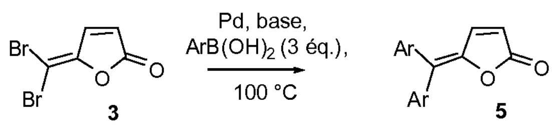


3
5

Les deux méthodes employées ont été comparées, les résultats sont rapportés ci-dessous.

| Lactones | Substituants | Méthode A Rendements (%) | Méthode B Rendements (%) |
|---|---|---|---|
| 4a | $C_6H_5$- | 74 | 55 |
| 4b | 3-thiényl- | 52 | 72 |
| 4c | naphtyl- | 63 | 35 |
| 4d | 3-MeO,4-MeO-$C_6H_3$- | 47 | -[a] |
| 4e | *p*-NBoc-$C_6H_4$- | 52 | 25 |
| 4f | *o*-F-$C_6H_5$- | - | 71 |
| 5a | $C_6H_5$- | 62 | 83 |
| 5b | 3-thiényl- | 66 | 40 |
| 5c | naphtyl- | 57 | 88 |
| 5d | 3-MeO,4-MeO-$C_6H_3$- | 50 | 25 |
| 5e | *p*-NBoc-$C_6H_4$- | - | 86 |
| 5f | *o*-F-$C_6H_5$- | - | 80 |
| 5h | *3,4,5-tri-MeO-* $C_6H_2$- | 70 | - |
| 5j | *4-MeS-* $C_6H_4$- | 60 | - |

a : absence de réaction
Rendements obtenus pour les couplages sur les lactones bromées 2 et 3

Cas des dérivés de méthylène furanone 4 et 5 substituées par des aromatiques différents

Monosubstitution régioséléctive des lactones bromées 2 et 3 par couplage de Suzuki-Miyaura

Nous avons étudié la régioséléctivité de cette réaction en couplant un seul groupe aryle par dérivé dibromé. Pour ce faire, nous avons utilisé les mêmes conditions opératoires (méthodes A et B) mais à température ambiante pour éviter la disubstitution et avec 1,1 équivalent d'acide boronique (l'utilisation d'un nombre d'équivalents supérieur mène majoritairement à une disubstitution). Dans chacun des cas testés, nous avons observé la formation d'un seul régioisomère (6 et 7).

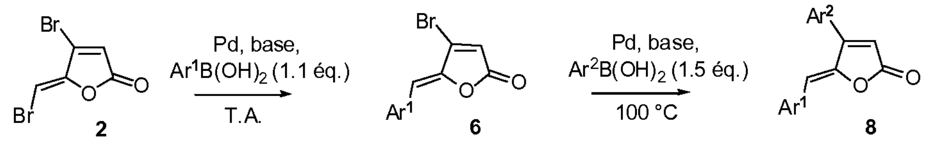


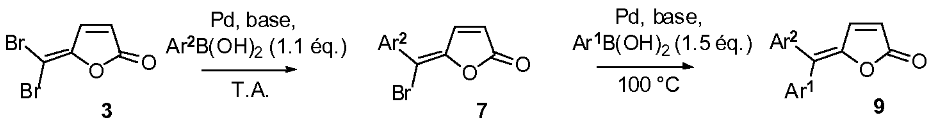


*Synthèse de dérivés de méthylène furanones par monosubstitutions successives*

Les rendements de cette réaction sont faibles à moyens ce qui semble principalement dû à une dégradation du produit de départ dans le milieu réactionnel. Effectivement dans la majorité des cas il n'en reste que des traces en fin de réaction. De plus, la formation de composés secondaires disubstitués est très faible, sauf pour **6b** (méthodes A et B) et **6a** (méthode B). Cette réaction apparaît donc assez délicate.

| Bromométhylène furanone | | Méthode A Rendements (%) | Méthode B Rendements (%) |
|---|---|---|---|
| 6a | 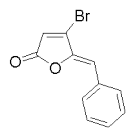 | 48 | 10 |

(suite)

| Bromométhylène furanone | | Méthode A Rendements (%) | Méthode B Rendements (%) |
|---|---|---|---|
| 6b | 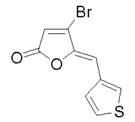 | 15 | 27 |
| 7a | 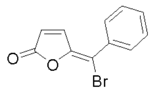 | 45 | 48 |
| 7b | 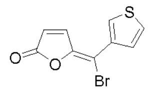 | 40 | |

*Rendements obtenus pour le mono-couplage sur les bromométhylène-furanones **2** et **3***

A partir des bromométhylène furanones **6** et **7**, le second couplage de Suzuki-Miyaura a été entrepris, en employant d'autres acides boroniques, de manière à obtenir les dérivés de méthylène furanone **8** et **9** substitués par deux aromatiques différents. Les conditions expérimentales utilisées se réfèrent toujours aux méthodes A et B avec cette fois 1,5 équivalent d'acide boronique à 100°C. Les rendements de cette réaction apparaissent également moyens, bien que la conversion des produits de départ soit totale.

| Dérivés de méthylène furanone | | Méthode A Rendements (%) | Méthode B Rendements (%) |
|---|---|---|---|
| 8a | 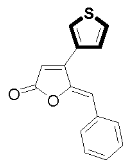 | 48 | |
| 8b | 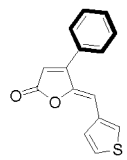 | | 66 |
| 9a | 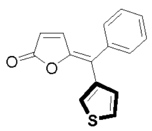 | 48 | |
| 9b | 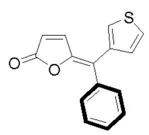 | 42 | |
| a : Les aromatiques en gras sont issus du second couplage. | | | |

**Détermination structurale par RMN**

Afin de déterminer la structure des composés issus d'un monocouplage régiosélectif, nous avons réalisé une étude RMN sur un composé de chaque série : série furanone disubstituée en 4 et 5 : composé **6a,** et série furanone gem-disubstituée en 5 : composé **9b.**

- série furanone disubstituée en 4 et 5 : composé **6a**

L'étude 2D proton-carbone HMBC nous a permis de déterminer facilement la régiochimie de la réaction lors du premier couplage organométallique et confirme que c'est bien la position 6 qui a été substituée.

- série furanone disubstituée 5 : composé **9b**

Nous avons tout d'abord effectué la comparaison des donnés RMN du composé 7a avec les données de la littérature (Sorg, A. Siegel, K. ; Brückner, R. Synlett 2004, 2, 231-325.). Celles-ci indiquent que nous serions en présence du stéréoisomère Z.. Pour le composé 9b nous avons réalisé une étude NOESY. Le composé 9b est issu d'un premier couplage avec un acide thiophèneboronique, puis d'un couplage avec l'acide phénylboronique. Nous observons bien une corrélation entre le proton $H_4$ et les protons du thiophène, ce qui confirme bien la stéréochimie Z du composé issu du premier couplage.

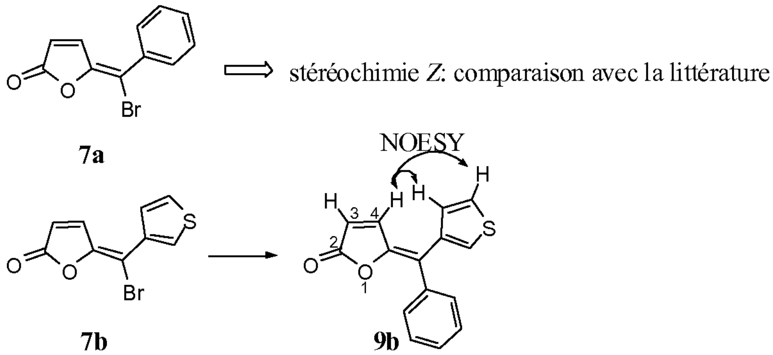

7a

7b 9b

2- Via 2-Méthoxy-furane

D'après la littérature, deux étapes : addition du lithien du 2-méthoxyfurane sur la cétone (JACS, 1986, 7055 et Synth. Comm., 2008, 212) puis déshydratation de l'alcool obtenu (JACS, 1986, 7055) :

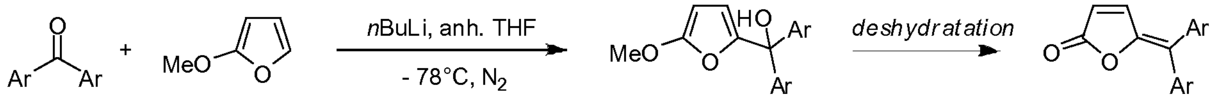


Cependant, la déshydratation est parfois spontanée et l'oléfine est alors directement isolée sans passer par l'alcool.

L'exemple connu dans la littérature est le suivant (JACS, 1986, 7055) :

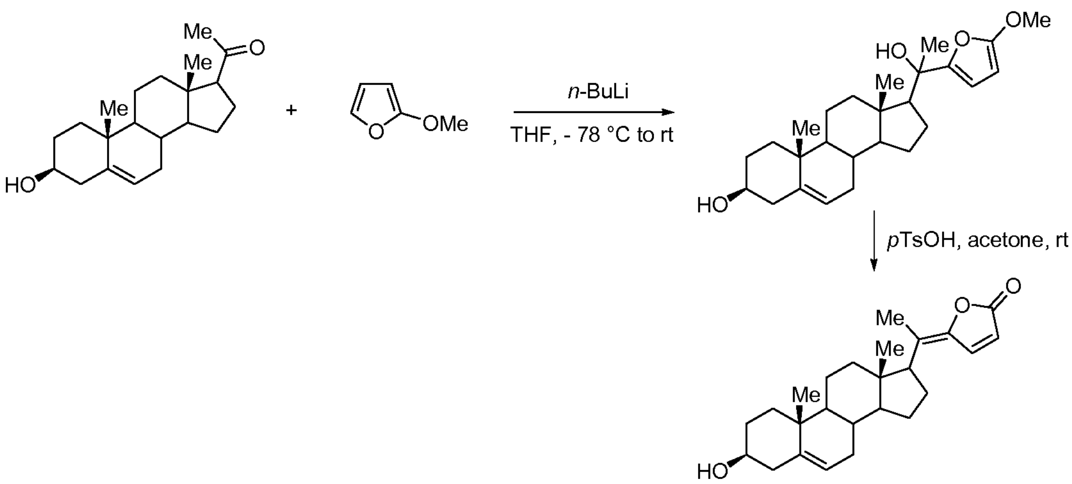


<u>- A partir de la 3',3',4',4'-Tetramethoxybenzophenone (10)</u>

La cétone de départ **10** est obtenue suivant les conditions décrites dans la littérature. (Eur. J. Org. Chem., 2004, 2381)

La cétone **10** a ensuite été engagée dans la réaction suivante pour donner directement l'oléfine **5d.** Le composé

**5d** a été traité avec du tribromure de bore (Tet., 2005, 2055) pour offrir le dérivé phénolique **5g** (73%) sous forme de solide vert.

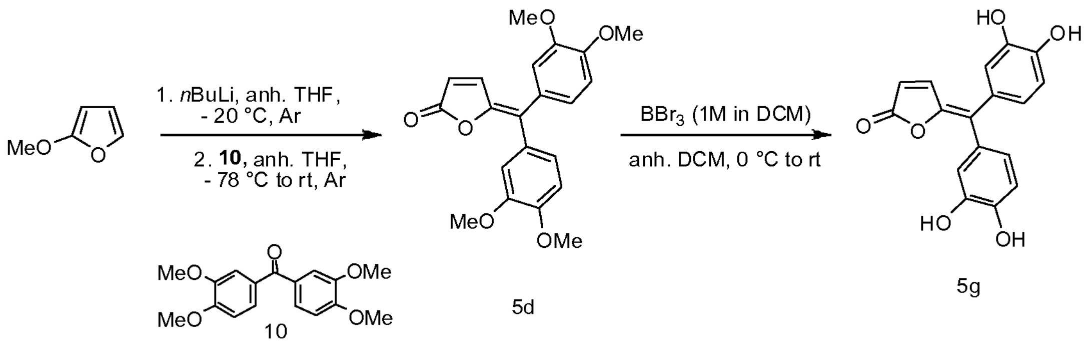


10

5d

5g

<u>- A partir de la di-2-thienylketone :</u>

Le composé a été obtenu en une seule étape avec un rendements de 52%, à partir du 2-méthoxyfurane et de la cétone di-2-thienyl.

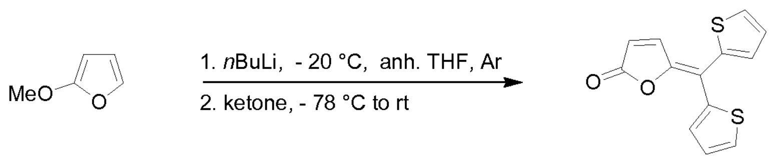


MG236F1 (322 mg, 28%)
MG240F2 (590 mg, 52%)

<u>- A partir de la di-3-thienylketone :</u>

Le composé **5b** (34%) a été obtenu en une seule étape à partir du 2-méthoxyfurane et de la cétone di-3-thienyl préparée suivant la procédure décrite (Synthesis 2000 1253)

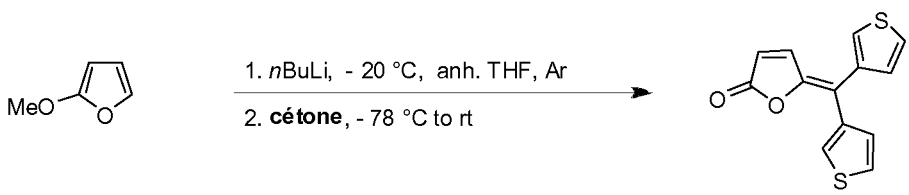


5b

La présente invention concerne donc également un procédé de préparation de dérivés de méthylène furanone de formule générale (I), sous leurs formes isomères (E) ou (Z), pures ou en mélange :

avec nécessairement $R_3$ représente un hydrogène,
dans lesquels $R_1$,et $R_2$, sont identiques et représentent :

- un radical phényle substitué par un ou plusieurs radicaux
    - ◦ alkyl($C_{1-5}$), alkyl($C_{1-4}$)oxy,
    - ◦ hydroxy,
    - ◦ méthylthio,
    - ◦ halogéno, ou
    - ◦ un groupement amino éventuellement substitué par un radical alkyl($C_{1-4}$), alkyl($C_{1-4}$)oxycarbonyle,
- un radical thiophényle,

- un radical thionaphtènyle,
- un radical thiazolyle,
- un radical pyridinyle
- un radical benzomorpholinyle,
- un radical naphtyle,

et
à la condition toutefois que $R_1$ et $R_2$ ne représentent pas simultanément un radical paraméthoxyphényle caractérisé en ce qu'il implique une réaction de couplage de Suzuki-Myaura à partir de dérivés dibromofuranones de formule générale (II), sous leurs formes isomères (E) ou (Z), pures ou en mélange :

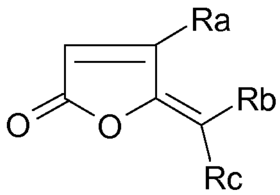

dans laquelle
Ra représente un hydrogène et Rb, Rc représente un radical bromo.
La présente invention concerne également un procédé de synthèse des dérivés de méthylène furanone de formule générale (I), sous leurs formes isomères (E) ou (Z), pures ou en mélange :

avec nécessairement $R_3$ représente un hydrogène,
dans lesquels $R_1$,et $R_2$, sont identiques et représentent :

- un radical phényle substitué par un ou plusieurs radicaux
  - alkyl($C_{1-5}$), alkyl($C_{1-4}$)oxy,
  - hydroxy,
  - méthylthio,
  - halogéno, ou
  - un groupement amino éventuellement substitué par un radical alkyl($C_{1-4}$), alkyl($C_{1-4}$)oxycarbonyle,
- un radical thiophényle,
- un radical thionaphtènyle,
- un radical thiazolyle,
- un radical pyridinyle
- un radical benzomorpholinyle,
- un radical naphtyle,

et
à la condition toutefois que $R_1$ et $R_2$ ne représentent pas simultanément un radical paraméthoxyphényle caractérisé en ce qu'il implique une réaction de couplage du 2-méthoxyfurane avec une cétone suivie d'une déshydratation in situ ou avec un agent de déshydratation.
La présente invention sera décrite ci-après plus en détail à la lumière des exemples de préparation indiqués ci-après à titre d'illustrations non limitatifs.

**Synthèse des intermédiaires**

- <u>acide 3,5-dibromolévulinique (1)</u> :

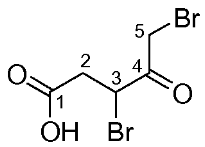

L'acide lévulinique (12 g, 0,103 mol) est dissous dans du chloroforme (60 mL). Le $Br_2$ (27.7 mL, 0.226 mol) est ajouté très lentement (2 h 30) à température ambiante. Du chloroforme (80 mL) est rajouté au milieu réactionnel pour aider à l'agitation. HBr est chassé par un courant d'azote.

Une heure et trente minutes après la fin de l'ajout de brome, le milieu réactionnel est refroidi à 0°C. Le précipité est filtré et lavé au chloroforme pour donner un solide blanc avec 53 % de rendement (15 g, 0,054 mol).

P.f. : 110-112 °C; CCM : (hexane/MeOH 50/50) $R_f$ = 0,25 ; $^1$H-RMN ($CDCl_3$) : $\delta$ = 3,05 (dd, 1H, $J$ = 17,8, 5,9 Hz, $H_2$), 3,38 (dd, 1H, $J$ = 17,8, 8,5 Hz, $H_2$), 4,17 (d, 1H, $J$ = 13,1 Hz, $H_5$), 4,38 (d, 1H, $J$ = 13,1 Hz, $H_5$), 5,01 (dd, 1H, $J$ = 8,5, 5,9 Hz, $H_3$) ppm.

- <u>4-bromo-5-(bromométhylène)furane-2(*5H*)-one (2) :</u>

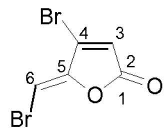

Une suspension d'acide 3,5-bromolévulinique **1** (1,00 g, 3,65 mmol) dans l'acide sulfurique (98 %, 5 mL) est agitée à 105°C pendant 20 min. Une fois revenue à température ambiante, la solution brune obtenue est versée sur de la glace, et extraite avec du $CH_2Cl_2$ (2 fois, 30 mL). Les phases organiques réunies sont lavées avec une solution d'eau saturée en NaCl et séchées sur $Na_2SO_4$. Les solvants sont évaporés à l'évaporateur rotatif, le résidu brun obtenu est chromatographié sur silice et élué avec un mélange pentane/$Et_2O$ (90/10). Des cristaux blancs crème sont obtenus avec 35 % de rendement (0,33 g, 128 mmol).

$R_f$ = 0,58 ($Et_2O$/pentane, 10:90), p. f. 97-98°C. $^1$H-RMN ($CDCl_3$) : $\delta$ = 6,50 (s, 1H, 3-H), 6,42 (s, 1 H, 6-H) ppm. $^{13}$C-RMN ($CDCl_3$) : $\delta$ = 93,7 (C-6), 121,0 (C-3), 135,3 (C-4), 165,4 (C-2) ppm. IR (KBr) : $\nu$ = 3129, 3080, 1809 (C=O), 1771 (C=O), 1642, 1553, 1552, 1438, 1311, 1247, 1167, 1118, 1085, 1006, 977, 928, 904, 845, 812, 788, 754, 718, 646, 538 cm$^{-1}$.

- <u>5-(dibromométhylène)furane-2(*5H*)one (3) :</u>

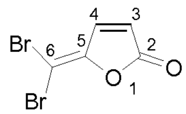

A l'acide 3,5-bromolévulinique 1 (2,37 g, 8,65 mmol) est ajouté un mélange d'oléum (acide sulfurique fumant à 65 % de $SO_3$, 4 mL) et d'acide sulfurique (98 %, 2 mL) à température ambiante. Après chauffage à 60-65°C pendant 6 min, le milieu réactionnel est ramené à température ambiante et versé sur 100 g de glace. Un précipité jaune apparaît et le mélange est extrait avec du $CH_2Cl_2$ (6 fois, 50 mL). Les phases organiques réunies sont lavées avec une solution d'eau saturée en NaCl, et séchées sur $Na_2SO_4$. Le solvant est évaporé à l'évaporateur rotatif, et le résidu brun obtenu est chromatographié sur silice avec comme éluant un mélange pentane/$Et_2O$ (75/25). Des cristaux blancs sont obtenus avec 31 % de rendement (0,29 g, 1,13 mmol).

$R_f$ = 0,64 ($Et_2O$/pentane, 25:75), p. f. 133-135°C. $^1$H-RMN ($CDCl_3$) : $\delta$ = 6,42 (d, 1H, $J$ = 5,4 Hz, 3-H). 7,68 (d, 1H, $J$= 5,6 Hz, 4-H) ppm. IR (KBr) : $\nu$ = 3128, 3099, 3065, 1890, 1786 (C=O), 1763 (C=O), 1651, 1611, 1547, 1455, 1345, 1257, 1124, 1097, 961, 892, 830, 774, 702, 529 cm$^{-1}$.

**Préparation du ligand S-Phos 10**

- 2-dicyclohexylphosphino-2',6'-diméthoxybiphényle :

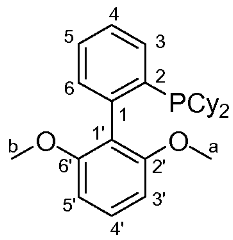

A une solution de 1,3-diméthoxybenzène (2 mL, 15,30 mmol) dans du THF anhydre (35 mL) à 0°C, est ajouté nBuLi (6,2 mL, 15,50 mmol) à l'ampoule à addition pendant 5 min. Le milieu réactionnel est agité à température ambiante pendant 3 h 30, puis le 2-bromochlorobenzène (1,6 mL, 13,70 mmol) est ajouté à la seringue, au goutte-à-goutte, à 0°C, pendant 30 min. Après 15 min d'agitation, le milieu réactionnel est refroidi à -78°C et nBuLi (6,20 mL, 15,50 mmol) ajouté à l'ampoule à addition au goutte-à-goutte pendant 5 min. Après 30 min, la chlorodicyclohexylphosphine (3,03 mL, 13,70 mmol) est ajoutée. Le milieu réactionnel est maintenu à -78°C pendant 1 h, sous vive agitation (agitation mécanique). Après retour à température ambiante, le précipité obtenu est filtré sur un fritté contenant de la silice surmontée d'une couche de Célite, avec 600 mL d'acétate d'éthyle. Les solvants sont évaporés à l'évaporateur rotatif, et l'huile orange obtenue est recristallisée dans l'acétone pour obtenir le ligand S-Phos sous forme de cristaux blancs avec 36 % de rendement (1,22 g, 2,97 mmol).
P.f. : 163-165°C (Lit.[32] p. f. 162,0-162,5°C) ; CCM : (AcOEt/cyclohexane 10/90). $R_f$ = 0,65 ; $^1$H-RMN ($CDCl_3$) : $\delta$ = 0,99-1,26 (m, 10H, H(Cy)), 1,60-1,77 (m, 12H, H(Cy)), 3,67 (s, 6H, Me), 6,58 (d, 2H, *J* = 8,2 Hz, $H_{3'}$ et $H_{5'}$), 7,15-7,18 (m, 1H, H(Ar)), 7,18-7,42 (m, 3H, H(Ar)), 7,57 (d, 1H, *J* = 7,4 Hz, H(Ar)) ppm ; $^{13}$C-RMN ($CDCl_3$) : $\delta$ = 26,5, 27,3, 27,4, 27,6, 29,0, 29,1, 29,8, 30,1, 33,8, 34,0 (C(Cy)), 55,3 (Cb), 103,1 (Ca), 126,2, 128,2, 128,8 ($C_{3'}$, $C_{4'}$, $C_{5'}$), 130,9, 131,00, 132,4, 135,8 ($C_3$, $C_4$, $C_5$, $C_6$), 135,8, 136,1, 142,7, 143,1, 157,4 ($C_{2'}$, $C_{6'}$) ppm; IR (KBr) : $\nu$ = 3000, 2923, 2851, 1588, 1471, 1442, 1428, 1108 $cm^{-1}$.

**Méthodes générales pour le couplage de Suzuki-Miyaura :**

**Méthode A** : Dans un tube de Schlenk, sous atmosphère inerte, contenant le dérivé bromé (150 mg, 0,59 mmol), l'acide boronique (1,77 mmol, 3,00 éq.), $Na_2CO_3$ (1,77 mmol, 3,00 éq.) et $Pd(PPh_3)_4$ (0,017mmol, 3 mol %) sont ajoutés du toluène (3 mL), $H_2O$ (1,5 mL) et de l'éthanol (0,75 mL). Le tube de Schlenk est ensuite bouché avec un septum, et le milieu réactionnel porté à 90°C pour 4 h. Après retour à température ambiante, de l'eau (30 mL) est ajoutée au milieu réactionnel. Il est extrait avec du $CH_2Cl_2$ (3 $\times$ 30 mL). Les phases organiques réunies sont lavées avec 30 mL d'une solution d'eau saturée en NaCl et séchées sur $Na_2SO_4$. Les solvants sont évaporés à l'évaporateur rotatif et le résidu est chromatographié sur silice.

**Méthode B :** Dans un tube de Schlenk, sous atmosphère inerte, contenant le dérivé bromé (150 mg, 0,59 mmol), l'acide boronique (1,77 mmol, 3,00 éq.), $K_3PO_4$ (1,77 mmol, 3,00 éq.), S-Phos 10 (0,012 mmol, 2 mol %) et $Pd(OAc)_2$ (0,006 mmol, 1 mol %) est ajouté du toluène (3 mL). Le tube de Schlenk est ensuite bouché avec un septum, et le milieu réactionnel porté à 100°C pour 4 h. Après retour à température ambiante, du $CH_2Cl_2$ (30 mL) est ajouté au milieu réactionnel. Il est filtré sur un fritté contenant de la silice. Les solvants sont évaporés à l'évaporateur rotatif et le résidu est chromatographié sur silice.

**Synthèse des composés**

- <u>(Z)-4-phényl-5-(phénylméthylène)furane-2(*5H*)-one (4a) :</u>

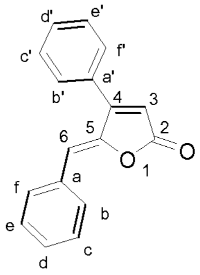

est préparé conformément aux méthodes A et B, et purifié sur silice avec comme éluant un mélange $CH_2Cl_2$/pentane (70/30), pour donner un solide blanc. Méthode A: 74 % de rendement (102 mg, 0,41 mmol) ; méthode B : 55 % de rendement (80 mg, 0,32 mmol).

$R_f$ = 0,58 ($CH_2Cl_2$/pentane, 80:20), p. f. 124-125°C. $^1$H-RMN ($CDCl_3$) : $\delta$ = 6,18 (s, 1H, 3-H ou 6-H), 6,21 (s, 1H, 3-H ou 6-H), 7,33 (t, 1H, *J* = 7,3 Hz, a-H), 7,39 (dd, 2H, *J* = 7,8 Hz, *J* = 7,3 Hz, c-H, e-H), 7,54-7,51 (m, 5H, b'-H, c'-H, d'-H, e'-H, f'-H), 7,81 (d, 2H, *J* = 7,8 Hz, b-H, f-H) ppm. $^{13}$C-RMN ($CDCl_3$) : $\delta$ = 113,9, 114,5 (C-3, C-6), 128,5, 128,8, 129,1, 129,3, 130,4, 130,5, 130,8, 132,9, 147,9 (C-5), 158,8 (C-4), 168,8 (C-2) ppm. IR (KBr) : $\nu$ = 3105, 3056, 1757 (C=O), 1645, 1606, 1585, 1568, 1491, 1449, 1348, 1309, 1218, 1184, 1158, 1088, 1075, 1029, 1000, 954, 914, 867, 840, 815, 757, 690, 658, 638 $cm^{-1}$. UV ($CH_2Cl_2$) : $\lambda_{max}$ ($\varepsilon$) = 238 (11 100), 339 nm (19 200 $Lmol^{-1}cm^{-1}$). SMRH (EI) : calculé pour $C_{17}H_{12}O_2$: 248,0837 ; trouvé 248,0832 ; $C_{17}H_{12}O_2$ (248,28) : C 82,24, H 4,87 ; trouvé C 82,06, H 4,96.

- <u>(Z)-4-(3-thiophène)-5-(3-thiophénylméthylène)furane-2(*5H*)one (4b) :</u>

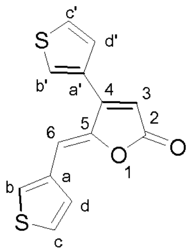

est préparé conformément aux méthodes A et B, et purifié sur silice avec comme éluant un mélange $CH_2Cl_2$/pentane (70/30), pour donner un solide jaune. Méthode A : 52 % de rendement (53 mg, 0,20 mmol) ; méthode B : 72 % de rendement (111 mg, 0,42 mmol).

$R_f$ = 0,49 ($CH_2Cl_2$/pentane, 70:30), p. f. 104-106°C. $^1$H-RMN ($CDCl_3$) : $\delta$ = 6,17 (d, 1H, *J* = 0,8 Hz, 6-H), 6,42 (s, 1H, 3-H), 7,29 (dd, 1H, *J*= 5,1 Hz, *J*= 1,3 Hz, c'-H), 7,36 (dd, 1H, *J* = 4,9 Hz, *J*= 2,8 Hz, d-H), 7,52 (dd, 1H, *J*= 5,0 Hz, *J*= 2,9 Hz, d'-H), 7,57 (dd, 1H, *J* = 5,1 Hz, *J*= 1,3 Hz, c-H), 7,64 (dd, 1H, *J*= 2,9 Hz, *J*= 1,4 Hz, b-H), 7,79-7,80 (m, 1H, b'-H) ppm. $^{13}$C-RMN ($CDCl_3$) : $\delta$ = 107,4 (C-6), 113,2 (C-3), 126,2, 126,5, 127,4, 127,5, 128,8, 129,0, 130,9, 134,4, 146,9 (C-5), 152,4 (C-4), 168,8 (C-2) ppm. IR (KBr) : $\nu$ = 3100, 3073, 1749 (C=O), 1740 (C=O), 1647, 1591, 1501, 1409, 1333, 1300, 1234, 1093, 979, 955, 934, 881, 867, 860, 831, 801, 785, 763, 688, 674, 628, 609, 602 $cm^{-1}$. UV ($CH_2Cl_2$) : $\lambda_{max}$ ($\varepsilon$) = 229 (13 100), 287 nm (12 300 $Lmol^{-1}cm^{-1}$). SMRH (EI): calculé pour $C_{13}H_8O_2S_2$: 259,9966 ; trouvé 259,9969 ; $C_{13}H_8O_2S_2$ (260,33) : C 59,98 ; H 3,10 ; trouvé C 59,90, H 3,13.

- <u>(Z)-4-(2-naphtalène)-5-(2-naphtalènephénylméthylène)furane-2(*5H*)-one (4c):</u>

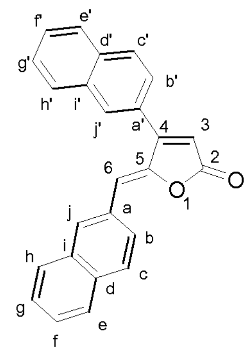

est préparé conformément aux méthodes A et B, et purifié sur silice avec comme éluant un mélange $CH_2Cl_2$/pentane (70/30), pour donner un solide jaune. Méthode A : 63 % de rendement (129 mg, 0,37 mmol) ; méthode B : le produit obtenu est recristallisé dans un mélange AcOEt/cyclohexane : 35 % de rendement (72 mg, 0,21 mmol).

$R_f$ = 0,46 ($CH_2Cl_2$/pentane, 70:30), p. f. 173-174°C. $^1$H RMN ($CDCl_3$) : δ = 6,32 (s, 1H, 3-H ou 6-H), 6,42 (s, 1H, 3-H ou 6-H),7,46-7,53 (m, 2H, ArH), 7,58-7,65 (m, 3H, ArH), 7,80-7,87 (m, 3H, ArH), 7,93-8,03 (m, 5H, ArH), 8,21 (s, 1H, ArH) ppm. $^{13}$C-RMN ($CDCl_3$) : δ = 114,3, 114,6 (C-3, C-6), 125,5, 126,5, 127,2, 127,2, 127,4, 127,7, 127,9, 128,5, 128,5, 128,6, 128,7, 129,0, 130,6, 131,2, 132,9, 133,3, 133,4, 133,9, 148,3 (C-5), 158,9 (C-4), 169,0 (C-2) ppm. IR (KBr) : ν = 3094, 3048, 1775 (C=O), 1643, 1628, 1607, 1581, 1565, 1506, 1371, 1343, 1315, 1272, 1187, 1130, 1082, 977, 942, 920, 898, 886, 856, 848, 821, 814, 790, 769, 740 $cm^{-1}$. UV ($CH_2Cl_2$) : $\lambda_{max}$ (ε) = 279 (21 000), 352 nm (22 000 $Lmol^{-1}cm^{-1}$). SMRH (EI) : calculé pour $C_{25}H_{16}O_2$ : 348,1150 ; trouvé 348,1136 ; $C_{25}H_{16}O_2$ (348,39) : C 86,19, H 4,63 ; trouvé C 85,98 ; H 4,80.

- <u>(Z)-5-(3,4-diméthoxyphénylméthylène)-4-(3,4-diméthoxyphényl-méthylène)furane-2(*5H*)-one (4d) :</u>

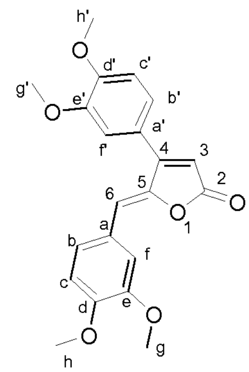

Méthode A : La synthèse est réalisée à partir d'1 g de (Z)-4-bromo-5-(bromométhylène)furane-2(*5H*)-one **2**. Le produit obtenu est purifié sur silice avec comme éluant un mélange $CH_2Cl_2$/AcOEt (93/7), puis recristallisé dans un mélange AcOEt/cyclohexane pour donner des cristaux verts avec 47 % de rendement (691 mg, 1,87 mmol).

$R_f$ = 0,66 ($CH_2Cl_2$/EtOAc, 93:7), p. f. 165-167°C. $^1$H-RMN ($CDCl_3$) : δ = 6.12 (s, 1H, 3-H), 6,18 (s, 1H, 6-H), 6,88 (d, 1H, *J* = 8,5 Hz, e-H), 6,99 (d, 1H, *J* = 1,8 Hz, b'-H), 7,01 (d, 1H, *J* = 8,2 Hz, e'-H), 7,12 (dd, 1H, *J* = 8,2 Hz, *J* = 2,1 Hz, f'-H), 7,32 (dd, 1H, *J* = 8,5 Hz, *J* = 2,1Hz, f-H), 7,48 (d, 1H, *J* = 2,1 Hz, b-H) ppm. $^{13}$C-RMN ($CDCl_3$) : δ = 55,9, 56,0, 56,1, 56,2, 111,0, 111,3, 111,5, 112,4, 112,6 (C-3), 112,9, 113,9, 114,0 (C-6), 121,6, 123,1, 124,9, 126,2, 146,1, 146,8 (C-5), 149,3, 150,3, 150,9, 158,5 (C-4), 169,2 (C-2) ppm. IR (KBr) : ν = 2935, 2837, 1749 (C=O), 1646, 1581, 1510, 1424, 1329, 1243, 1167, 1144, 1025, 930, 876, 811, 765, 750, 597 $cm^{-1}$. UV ($CH_2Cl_2$): $\lambda_{max}$ (ε) = 263 (16 400), 373 nm (20 700 $Lmol^{-1}cm^{-1}$). SMRH (EI) : calculé pour $C_{21}H_{20}O_6$ : 368,1259 ; trouvé 368,1248 ; $C_{21}H_{20}O_6$ (368,38) : C 68,47, H 5,47 ; trouvé C 67,31, H 5,49.

- <u>(Z)-4-(hydroxyphénylcarbamate de *tert*-butyl)(Z)-5-(hydroxyphényl-carbamate de *tert*-butylmélène)furane-2(*5H*)-one (4e) :</u>

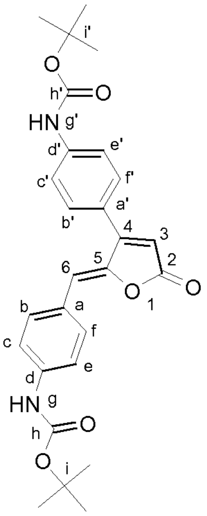

est préparé conformément aux méthodes A et B, et purifié sur silice avec comme éluant un mélange $Et_2O$/pentane (40/60), pour donner un solide jaune. Méthode A : 52 % de rendement (148 mg, 0,31 mmol) ; méthode B: le produit obtenu est recristallisé dans $CH_2Cl_2$: 25 % de rendement (47 mg, 0,10 mmol).

$R_f$ = 0,68 ($Et_2O$/pentane, 60:40), p. f. 140-142°C déc. $^1$H-RMN ($CDCl_3$) : $\delta$ = 1,53 (s, 9H, $CH_3$), 1,55 (s, 9H, $CH_3$), 6,13 (s, 1H, 3-H ou 6-H), 6,14 (s, 1H, 3-H ou 6-H), 6,62 (s, 1H, h-H ou h'-H), 7,41 (d, 2H, *J*= 8,9 Hz, b-H, f-H ou b'-H, f'-H), 7,45 (d, 2H, *J* = 8,9 Hz, b-H, f-H ou b'-H, f'-H), 7,53 (d, 2H, *J* = 8,6 Hz, c-H, e-H ou c'-H, e'-H), 7,75 (d, 2H, *J*= 8,6 Hz, b-H, f-H ou b'-H, f'-H) ppm. $^{13}$C-RMN ($CDCl_3$) : $\delta$ = 28,3 ($CH_3$), 81,0, 81,4, 112,8 (C-6 ou C-3), 113,6 (C-6 ou C-3), 118,2, 118,6, 124,9, 127,8, 129,4, 131,9, 139,4, 140,6 147,1 (C-5), 152,4, 152,5, 158,3 (C-4), 169,2 (C-2) ppm. IR (KBr) : $\nu$ = 3298, 2982, 1737 (C=O), 1694 (C=O), 1592, 1522, 1413, 1319, 1238, 1158, 1054, 956, 930, 838, 522 $cm^{-1}$. UV ($CH_2Cl_2$): $\lambda_{max}$ ($\varepsilon$) = 249 (25000), 351 nm (20 600 $Lmol^{-1}cm^{-1}$). SMRH (EI) : calculé pour $C_{27}H_{30}N_2O_6$: 378,1580 ; trouvé 378,1587 ($M-CO_2-C_4H_8^+$) ; $C_{27}H_{30}N_2O_6$ (478,54) : C 67,77, H 6,32, N 5,85 ; trouvé C 67,50, H 6,57, N 5,61.

- <u>(Z)-4-(2-fluorophényl)-(Z)-5-(2-fluorophénylméthylène)furane-2(*5H*)-one</u> (4f):

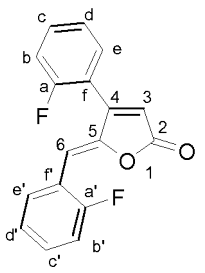

Méthode B : Le produit obtenu est purifié sur silice avec comme éluant un mélange $CH_2Cl_2$/pentane (50/50), pour donner un solide blanc avec 71 % de rendement (119 mg, 0,42 mmol).

$R_f$ = 0,48 ($CH_2Cl_2$/pentane, 50:50), p. f. 151-153°C. $^1$H-RMN ($CDCl_3$) : $\delta$ = 6,20 (s, 1H, 3-H), 6,83 (s, 1H, 6-H), 7,29-7,46 (m, 5H, ArH), 7,63-7,65 (m, 2H, ArH), 8,08-8,14 (m, 1H, H ArH) ppm. $^{13}$C-RMN ($CDCl_3$) : $\delta$ = 104,5 (d, $J^3_{F-C}$ = 8 Hz, C-6), 115,3 (d, $J^2_{F-C}$ = 22 Hz, C-b ou C-b'), 116,7 (d, $J^2_{F-C}$ = 22 Hz, C-b ou C-b'), 117,6 (d, $J^3_{F-C}$ = 3 Hz, C-c ou C-c' ou C-e ou C-e'), 117,9 (d, $J^2_{F-C}$ = 15 Hz, C-f ou C-f' ), 124,6 (C-3), 124,7 (d, $J^3_{F-C}$ = 3 Hz, C-c ou C-c' or C-e ou C-e'), 130,5 (d, $J^4_{F-C}$ = 2 Hz, C-d ou C-d'), 131,00 (d, $J^3_{F-C}$ = 9 Hz, C-c ou C-c' or C-e ou C-e'), 131,6 (d, $J^4_{F-C}$ = 1 Hz, C-d ou C-d'), 132,3 (d, $J^3_{F-C}$ = 8 Hz, C-c ou C-c' ou C-e ou C-e'), 150,3, 152,1 (C-4, C-5), 158,34 (d, $J^1_{F-C}$ = 78 Hz, C-a ou C-a'), 162,1 (d, $J^1_{F-C}$ = 80 Hz, C-a ou C-a'), 168,5 (C-2) ppm. IR (KBr) : $\nu$ = 3104, 1777 (C=O), 1621, 1573, 1481, 1455, 1343, 1235, 1208, 1116, 1079, 964, 927, 873, 848, 825, 805, 793, 760, 750, 679, 462 $cm^{-1}$. UV ($CH_2Cl_2$) : $\lambda_{max}$ ($\varepsilon$) = 238 (15 000), 341 nm (30 300 $Lmol^{-1}cm^{-1}$). SMRH (EI) : calculé pour $C_{17}H_{10}F_2O_2$: 284,0649 ; trouvé 284,0628 ; $C_{17}H_{10}F_2O_2$ (284,06) : C 71,83, H 3,55 ; trouvé C 71,60, H 3,62.

- 5-(diphénylméthylène)furane-2(*5H*)-one (5a) :

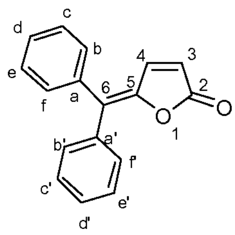

Le brut réactionnel obtenu est purifié sur gel de silice avec comme éluant un mélange $CH_2Cl_2$/pentane (70/30), pour donner un solide blanc. Méthode A : 62 % de rendement (90 mg, 0,36 mmol) ; méthode B : 83 % de rendement (162 mg, 0,65 mmol).
$R_f$ = 0,51 ($CH_2Cl_2$/pentane, 70:30), p. f. 110-112°C. $^1$H-RMN ($CDCl_3$) : $\delta$ = 6,21 (d, 1H, *J* = 5,4 Hz, 3-H), 7,26-7,52 (m, 11H, 4-H, b-H, c-H, d-H, e-H, f-H, b'-H, c'-H, d'-H, e'-H, f'-H) ppm. $^{13}$C-RMN ($CDCl_3$) : $\delta$ = 118,6 (C-3), 128,2, 128,5, 128,9, 129,1, 131,2, 136,5, 137,9 (C-6), 143,9 (C-4), 147,12 (C-5), 170,5 (C-2) ppm. IR (KBr) : $\nu$ = 3096, 1767 (C=O), 1745 (C=O), 1546, 1490, 1443, 1230, 1114, 1085, 957, 932, 877, 823, 774, 719, 697 $cm^{-1}$. UV ($CH_2Cl_2$) : $\lambda_{max}$ ($\varepsilon$) = 245 (13 500), 344 nm (22 200 $Lmol^{-1}cm^{-1}$). SMRH (EI) : calculé pour $C_{17}H_{12}O_2$ : 248,0837 ; trouvé 248,0835 ; $C_{17}H_{12}O_2$ (248,28) : C 82,24, H 4,87 ; trouvé : C 82,08, H 4,92.

- 5-(dithiophène-3-ylméthylène)furane-2(*5H*)-one (5b) :

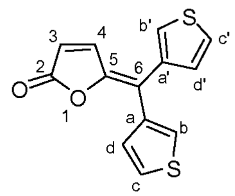

est préparé conformément aux méthodes A et B, et purifié sur silice avec comme éluant un mélange $CH_2Cl_2$/pentane (70/30), pour donner un solide jaune clair. Méthode A : 66 % de rendement (338 mg, 1,3 mmol) ; méthode B : est préparé à partir de 500 mg de 5-(dibromométhylène)furane-2(*5H*)one **3,** après colonne le produit est recristallisé dans un mélange $CH_2Cl_2/Et_2O$) : 40 % de rendement (205 mg, 0,78 mmol).
$R_f$ = 0,50 ($CH_2Cl_2$/pentane, 70:30), p. f. 122-124°C. $^1$H-RMN ($CDCl_3$) : $\delta$ = 6,16 (d, 1H, *J* = 5,4 Hz, 3-H), 7,10 (dd, 1H, *J* = 4,9 Hz, *J* = 1,3 Hz), 7,32-7,35 (m, 2H,), 7,43 (dd, 1H, *J* = 4,9 Hz, *J* = 3,1 Hz), 7,46 (d, 1H, *J* = 5,4 Hz, 4-H), 7,52 (dd, 1H, *J*= 5,1 Hz, *J* = 1,3 Hz), 7,55 (dd, 1H *J*= 2,8 Hz, *J*= 1,3 Hz) ppm. $^{13}$C-RMN ($CDCl_3$) : $\delta$ = 117,8 (C-3), 118,0 (C-6), 125,5, 126,2, 126,7, 129,4, 129,6, 129,8, 136,7, 137,3, 143,6 (C-4), 146,4 (C-5), 170,2 (C-2) ppm. IR (KBr) : $\nu$ = 3109, 1761 (C=O), 1740 (C=O), 1608, 1540, 1507, 1420, 1293, 1234, 1197, 1158, 1113, 1069, 994, 978, 909, 894, 841, 745, 705, 685, 620, 521 $cm^{-1}$. UV ($CH_2Cl_2$) : $\lambda_{max}$ ($\varepsilon$) = 229 (15 300), 359 nm (24 900 $Lmol^{-1}cm^{-1}$). SMRH (EI) : calculé pour $C_{13}H_8O_2S_2$ : 259,9966 ; trouvé 259,9969 ; $C_{13}H_8O_2S_2$ (260,33) :C 59,98, H 3,10 ; trouvé C 59,89, H 3,05.

- 5-(dinaphtalène-2-ylméthylène)furane-2(*5H*)-one (5c) :

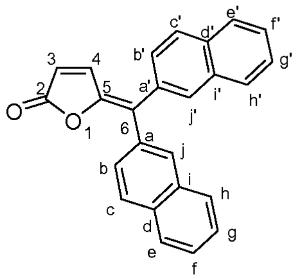

est préparé conformément aux méthodes A et B, et purifié sur silice avec comme éluant un mélange $CH_2Cl_2$/pentane (30/70), pour donner un solide jaune. Méthode A : 57 % de rendement (56 mg, 0,33 mmol) ; méthode B : 88 % de rendement (182 mg, 0,52 mmol) ;
$R_f$ = 0,63 ($CH_2Cl_2$/pentane, 70:30), p. f. 138-140°C. $^1$H-RMN ($CDCl_3$) : δ = 6,25 (d, 1H, *J* = 5,6 Hz, 3-H), 7,33-7,93 (m, 15H) ppm. $^{13}$C-RMN ($CDCl_3$) : δ = 118,6 (C-3, C-6), 126,4, 126,9, 127,1, 127,2, 127,6, 127,8, 128,1, 128,2, 128,3, 128,5, 128,9, 130,8, 131,7, 132,9, 132,9, 133,3, 133,3, 134,0, 134,7, 144,0 (C-4), 147,7, (C-5), 170,5 (C-2) ppm. IR (KBr) : ν = 3054, 1777 (C=O), 1752 (C=O), 1597, 1544, 1503, 1357, 1244, 1190, 1109, 938, 888, 821, 749, 709, 476 $cm^{-1}$. UV ($CH_2Cl_2$) : $\lambda_{max}(\varepsilon)$ = 230 (48 600), 238 (49 800), 256 (45 900), 363 nm (27 600 $Lmol^{-1}cm^{-1}$). SMRH (EI) : calculé pour $C_{25}H_{16}O_2$ : 348,1150 ; trouvé 348,1140. $C_{25}H_{16}O_2$ (348,39) : C 86,19, H 4.63 ; trouvé C 86,03, H 4,75.

- 5-(bis(3,4-diméthoxyphényl)méthylène)furane-2(*5H*)-one (5d) :

- Voie couplage Suzuki- Myaura

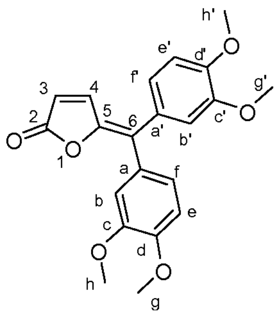

Le composé (5d) est préparé conformément aux méthodes A et B, et purifié sur silice avec comme éluant un mélange $CH_2Cl_2/Et_2O$ (92/8), pour donner une huile jaune. Méthode A : 50 % de rendement (107 mg, 0,29 mmol) ; méthode B : 25 % de rendement (51 mg, 0,14 mmol).
$R_f$ = 0,52 ($CH_2Cl_2/Et_2O$, 92:8), Rf (EtOAc/cyclohexane 1:2) = 0.21 p. f. 160-162°C. $^1$H-RMN ($CDCl_3$): δ = 3,83 (s, 3H, g-H, h-H, g'-H, ou h'-H), 3,84 (s, 3H, g-H, h-H, g'-H, ou h'-H), 3,92 (s, 3H, g-H, h-H, g'-H, ou h'-H), 3,96 (s, 3H, g-H, h-H, g'-H, ou h'-H), 6,15 (d, 1H, *J*= 5,4 Hz, 3-H), 6,76 (d, 1H, *J*= 1,8 Hz, b'-H), 6,85 (d, 1H, *J* = 8,5 Hz, e-H), 6,86-6,95 (m, 2H, f-H, e'-H), 7,07 (dd, 1H, *J* = 8,5 Hz, *J* = 2,0 Hz, f-H), 7,17 (d, 1H, *J* = 2,0 Hz, b-H), 7,41 (d, 1H, *J*= 5,4 Hz, 4-H) ppm. $^{13}$C-RMN ($CDCl_3$) : δ = 55,9, 56,0, 56,0, 110,7, 110,7, 114,2, 114,3, 117,3 (C-3), 124,3, 125,3, 128,6 (C-6), 129,3, 129,8, 114,0 (C-4), 146,3 (C-5), 148,5, 148,8, 149,8, 150,1, 170,7 (C-2) ppm. IR (KBr) : ν = 3132, 3107, 3059, 2999, 2957, 2934, 2910, 2836, 1778 (C=O), 1747 (C=O), 1595, 1577, 1538, 1511, 1462, 1413, 1349, 1321, 1306, 1252, 1239, 1211, 1171, 1140, 1111, 1069, 1023, 999, 981, 895, 862, 810, 761, 734, 710, 688 $cm^{-1}$. UV ($CH_2Cl_2$): $\lambda_{max}$ (ε) = 263 (17 700), 384 nm (19 900 $Lmol^{-1}cm^{-1}$). SMRH (EI) : calculé pour $C_{21}H_{20}O_6$ : 368,1260; trouvé 368,1248 ; $C_{21}H_{20}O_6$ (368,38) : C 68,47, H 5,47 ; trouvé C 68,28, H 5,64.

- Voie couplage de 2-methoxyfurane avec une cétone:

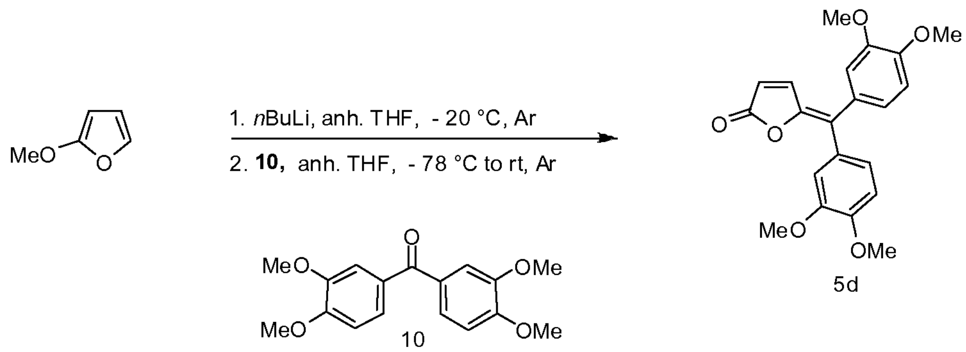


n-BuLi (2,5M dans hexane, 3,44 mL, 8,64 mmol) est ajouté à une solution de 2-méthoxyfurane (0.80 mL, 8.64 mmol, 1.3 eq.) dans 30 mL de THF anhydre à -20°C et sous argon. La solution jaune pâle est agitée 1H30 à -20°C. La solution orange obtenue est alors refroidie à -78°C et une solution de la cétone 10 (2.00 g, 6.61 mmol, 1.0 eq., *Eur. J. Org. Chem.*, **2004,** 2381) dans 40 mL de THF anhydre est ajoutée goutte à goutte. La bain est alors retiré et le mélange est agité à température ambiante pour la nuit.

La réaction est stoppée par addition d'une solution d'HCl 1N et laissée agitée pour une heure. Le produit est ensuite extrait à l'acétate d'éthyle.

Le solvant est évaporé pour conduire à un solide. Après lavage à l'éther, le produit est obtenu sous la forme d'un solide jaune avec un rendement de 83% (2,04 g).

- 5-(bis(4-hydroxyphénylcarbamate de *tert*-butyl)furane-2(*5H*)-one (5e) :

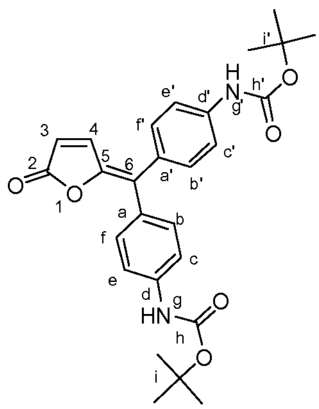

Méthode B : Est préparé à partir de 200 mg de 5-(dibromométhylène)furane-2(*5H*)one **3**. Le produit obtenu est purifié sur silice avec comme éluant un mélange $CH_2Cl_2$/AcOEt (90/10), pour donner un solide jaune avec 86 % de rendement (319 mg, 0,67 mmol).

$R_f$ = 0,51 ($CH_2Cl_2$/EtOAc, 95:5), p. f. 138-140°C. $^1$H-RMN ($CDCl_3$) : $\delta$ = 1,52 (s, 9H, O-$CH_3$), 1,54 (s, 9H, O-$CH_3$), 6,16 (d, 1H, *J* = 5,4 Hz, 3-H), 6,67 (s, 1H, N-H), 6,70 (s, 1H, N-H), 7,16 (s, 1H), 7,19 (s, 1H), 7,34-7,46 (m, 7H), 7,40 (d, 1H, *J* = 5,4 Hz, 4-H) ppm. $^{13}$C-RMN ($CDCl_3$) : $\delta$ = 28,3 ($CH_3$), 80,9, 81,0, 117,5 (C-3), 117,8, 118,2, 128,3 (C-6), 131,1, 131,6, 132,1, 132,4, 139,2, 139,25, 143,8 (C-4), 146,5 (C-5), 152,4, 152,6, 170,8 (C-2) ppm. IR (KBr) : $\nu$ = 3322 (N-H), 2977, 1773 (C=O), 1735 (C=O), 1708 (C=O), 1586, 1521, 1408, 1392, 1367, 1315, 1232, 1157, 1052, 1016, 961, 923, 882, 842, 809, 771, 708 $cm^{-1}$. UV ($CH_2Cl_2$): $\lambda_{max}$ ($\varepsilon$) = 259 (22 600), 377 nm (24 900 $Lmol^{-1}cm^{-1}$). $C_{27}H_{30}N_2O_6$ (478,54) : C 67,77, H 6,32, N 5,85 ; trouvé C 67,61, H 6,41, N 5,84.

- 5-bis((2-fluorophényl)méthylène)furane-2(*5H*)-one (5f) :

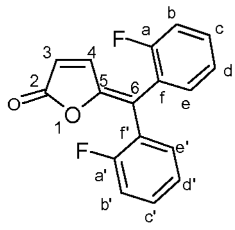

Méthode B : Le produit obtenu est purifié sur silice avec comme éluant un mélange $CH_2Cl_2$/pentane (70/30), pour donner un solide blanc avec 80 % de rendement (122 mg, 0,43 mmol).
$R_f$ = 0,45 ($CH_2Cl_2$/pentane, 70:30), p. f. 138-139°C. $^1$H-RMN ($CDCl_3$) : δ = 6,29 (d, 1 H, *J* = 5,6 Hz, 3-H), 7,05-7,44 (m, 9H) ppm. $^{13}$C-RMN ($CDCl_3$) : δ = 116,0 (d, $J^2_{F-C}$ = 46 Hz, C-b ou C-b'), 116,3 (d, $J^2_{F-C}$ = 46 Hz, C-b ou C-b'), 120,7 (C-3), 124,1 (dd, $J^2_{F-C}$ = 82 Hz, $J^4_{F-C}$ = 14 Hz, C-f ou C-f'), 130,8 (d, $J^3_{F-C}$ = 29 Hz, C-c, C-e, C-c', C-e'), 130,9 (d, $J^3_{F-C}$ = 27 Hz, C-c, C-e, C-c', C-e'), 131,7 (d ,$J^4_{F-C}$ = 11 Hz, C-d ou C-d'), 132,5 (d, $J^4_{F-C}$ = 8 Hz, C-d ou C-d'), 142,4 (C-6), 142,4 (C-4), 149,0 (C-5), 158,3 (d, $J^1_{F-C}$ = 91 Hz, C-a ou C-a'), 162,0 (d, $J^1_{F-C}$ = 80 Hz, C-a ou C-a'), 169,5 (C-2) ppm. IR (KBr) ν = 3146, 3123, 3078, 3039, 1779 (C=O), 1752 (C=O), 1641, 1608, 1577, 1552, 1485, 1444, 1258, 1244, 1225, 1183, 1117, 1074, 1033, 971, 958, 886, 813, 771, 742, 647, 621, 538 $cm^{-1}$. UV ($CH_2Cl_2$) : $\lambda_{max}$ (ε) = 244 (15 300), 328 nm (19 300 $Lmol^{-1}cm^{-1}$). SMRH (EI): calculé pour $C_{17}H_{10}O_2F_2$ : 284,0649; trouvé 284,0628 ; $C_{17}H_{10}O_2F_2$ (284,26) : C 71,83, H 3,55 ; trouvé C 71,66, H, 3,67.

- 5-(bis(3,4,5-trimethoxyphenyl)methylene)furan-2(5H)-one (5h) :

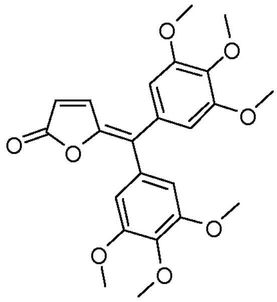

Le composé 5h est préparé conformément à la méthode A et purifié sur silice avec comme éluant un mélange Cyclohexane/Acétate d'éthyle (1/1), pour donner un solide jaune. Avec un rendement de 70 % de rendement.
Rf (EtOAc/cyclohexane 1:1) = 0.51
HRMS calc. for $M^+$ 428.1471, found 428.1471.
$\delta_H$ (270 MHz, $CDCl_3$): 7.46 (1H, d, *J* = 5.38 Hz, ,1H olefinic), 6.78 (2H, s, 2H aromatic), 6.51 (2H, s, 2H aromatic), 6.20 (1H, d, *J* = 5.38 Hz, 1H olefinic), 3.93 (3H, s, *OMe),* 3.90 (3H, s, *OMe),* 3.84 (6H, s, 2 x O*Me*), 3.81 (6H, s, 2 x O*Me*).
$\delta_C$ (67.80 MHz, $CDCl_3$): 170.24, 153.03, 152.76, 146.78, 143.95, 132.32, 131.50, 128.35, 118.04, 109.07, 108.78, 56.31.

- 5-(bis(3,4-dihydroxyphenyl)methylene)furan-2(5H)-one (5g) :

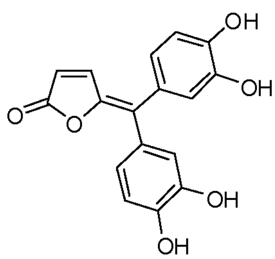

A une solution du composé 5d (400 mg, 1,08 mmol) dans 15 mL de dichlorométhane anhydre sous argon, est ajouté le BBr3 (1M dans DCM, 10,86 mL, 10,86 mmol, 10 eq) à 0°C. La solution rouge est alors agitée 1 heure à 0°C puis la réaction est stoppée par addition d'une solution d'HCl 1N puis la phase aqueuse est extraite à l'acétate d'éthyle. La phase organique est lavée à l'eau, puis par une solution saturée en NaCL et enfin séchée sur MgSO4. Le solvant est évaporé puis le solide obtenu est lavé à l'ether et séché sous vide pour conduire à un solide vert pale avec un rendement de 73% (249 mg)
Rf (EtOAc/MeOH 95:5) = 0.75
HRMS calc. for $M^+$ 366.1943
$\delta_H$ (270 MHz, acetone): 8.39 (1H, bs, 1 x O*H*), 8.37 (1H, bs, 1 x O*H*), 8.29 (1H, bs, 1 x O*H*), 8.26 (1H, bs, 1 x O*H*), 7.56 (1H, d, *J* = 5.44 Hz, *H* furan), 7.09 (1H, *d, J* = 1.86 Hz, *H* aromatic), 6.92 (1H, d, *J* = 8.05 Hz, *H* aromatic), 6.87-6.86 (2H, m, H aromatic), 6.76 (1H, *d, J* = 2.01 Hz, H aromatic), 6.67 (1H, dd, *J* = 2.08 and 8.05 Hz, H aromatic), 6.21 (1H, *d, J* = 5.41 Hz, *H* furan).
$\delta_C$ (67.80 MHz, acetone): 172.14, 148.37, 147.99, 147.67, 146.76, 146.35, 146.29, 130.99, 130.85, 130.46, 126.00, 125.22, 120.15, 120.12, 118.29, 116.89, 116.75.

- <u>5-(bis(3,5-trihydroxyphenyl)methylene)furan-2(5H)-one (5i) :</u>

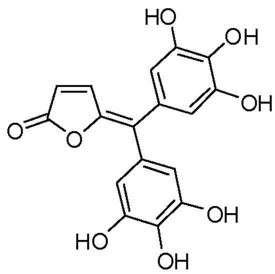

Le composé 5i est préparé conformément à la méthode utilisée pour la synthèse du composé 5g en partant de 200 mg de 5h avec un rendement de 93 % (159 mg, solide bleu vert)
**Rf** (EtOAc/MeOH 90:10) = 0.70;
**HRMS** calc. for $M^+$ 344.0532,
$\delta_H$ (270 MHz, acetone): 8.12 (3H ,d ,3 x O*H*), 7.73 (1H, d, 1 x O*H*), 7.56 (1H, d, *J*= 5.38 Hz, 1H olefinic), 6.64 (2H, s, 2H aromatic), 6.33 (2H, s, 2H aromatic), 6.17 (1H, d, *J*= 5.63 Hz, 1H olefinic), 2.19 (1H, bs, 1 x *OH*);
$\delta_C$ (67.80 MHz, acetone): 171.16, 146.65, 146.18, 145.82, 145.46, 135.35, 134.57, 129.62, 129.17, 128.71, 117.04, 111.83, 111.55.

- <u>(Z)-4-bromo-5-(phénylméthylène)furane-2(*5H*)-one (6a) :</u>

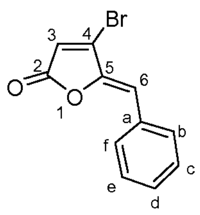

Dans un tube de Schlenk, sous atmosphère inerte, contenant 2 (200 mg, 0,78 mmol), l'acide phénylboronique (122 mg, 1,18 mmol), $Na_2CO_3$ (167 mg, 1,57 mmol) et $Pd(PPh_3)_4$ (27 mg, 3 mol %), sont ajoutés du toluène (4 mL), $H_2O$ (2 mL) et de l'éthanol (1 mL). Le tube de Schlenk est ensuite bouché avec un septum, et le milieu réactionnel est agité pendant 2 jours à température ambiante. De l'eau (10 mL) est ensuite ajoutée, et le milieu réactionnel est extrait avec de l'éther (3 $\times$ 30 mL). Les phases organiques réunies sont lavées avec une solution d'eau saturée en NaCl et séchées sur $Na_2SO_4$. Les solvants sont évaporés à l'évaporateur rotatif, et le résidu est chromatographié sur silice, avec comme éluant un mélange $Et_2O$/pentane (5/95). Un solide jaune orange est obtenu avec 47 % de rendement (94 mg, 0,37 mmol).

$R_f$ = 0,84 ($CH_2Cl_2$/pentane, 50:50), p. f. 68-70°C. $^1$H-RMN ($CDCl_3$) $\delta$ = 6,37 (s, 1H, 6-H), 6,42 (s, 1H, 3-H), 7,37-7,46 (m, 3H, c-H, d-H, e-H), 7,80 (d, 1H, *J* = 1,3 Hz, b-H ou f-H), 7,83 (d, 1H, *J* = 1,6 Hz, b-H ou f-H) ppm. $^{13}$C-RMN ($CDCl_3$): $\delta$ = 113,5 (C-6), 118,8 (C-3), 129,0, 129,9, 131,0, 138,5, 138,5 (C-5), 146,6 (C-4), 167,1 (C-2) ppm. IR (KBr) : $\nu$ = 3503, 3137, 3051, 3023, 2925, 1761 (C=O), 1645 (C=C), 1595, 1550, 1525, 1490, 1447, 1352, 1322, 1300, 1285, 1214, 1179, 1105, 1075, 1032, 999, 968, 911, 866, 848, 815, 753, 687, 668, 626, 613, 528 $cm^{-1}$. UV ($CH_2Cl_2$): $\lambda_{max}$ ($\varepsilon$) = 341 nm (29 800 $Lmol^{-1}cm^{-1}$). $C_{11}H_7O_2Br$ (251,08) : C 52,62, H 2,81 ; trouvé C 52,82, H 3,02.

- (Z)-5-(phénylméthylène)-4-(thiophène-3-yl)furane-2(*5H*)-one (8a) :

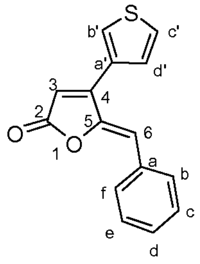

Dans un tube de Schlenk, sous atmosphère inerte, contenant **6a** (94 mg, 0,37 mmol), l'acide thiophène-3-boronique (0,71 mg, 0,56 mmol), $Na_2CO_3$ (79 mg, 0,75 mmol), $Pd(PPh_3)_4$ (13 mg, 3 mol %), sont ajoutés du toluène (3 mL), $H_2O$ (1,5 mL) et de l'éthanol (0,75 mL).

Le tube de Schlenk est ensuite bouché avec un septum, et le milieu réactionnel porté à 90°C pendant 6 h. Après retour à température ambiante, de l'eau (30 mL) est ajoutée au milieu réactionnel. Il est extrait avec du $CH_2Cl_2$ (3 × 20 mL). Les phases organiques réunies sont lavées avec 20 mL d'une solution d'eau saturée en NaCl et séchées sur $Na_2SO_4$. Les solvants sont évaporés à l'évaporateur rotatif, et le résidu est chromatographié sur silice avec comme éluant un mélange $Et_2O$/pentane (10/90). Un solide rose pâle est obtenu avec 48 % de rendement (45 mg, 0,18 mmol). $R_f$ = 0,55 ($Et_2O$/pentane, 20:80), p. f. 87-89°C. $^1$H-RMN ($CDCl_3$) : $\delta$ = 6,21 (s, 1H, 3-H), 6,34 (s, 1H, 6-H), 7,31 (dd, 1H, *J* = 4,9 Hz, *J* = 1,3 Hz, c'-H), 7,39 (m, 3H, c-H, d-H, e-H), 7,53 (dd, 1H, *J*= 5,0 Hz, *J*= 2,9 Hz, d'-H),7,65 (dd, 1H, *J*= 2,9 Hz, *J* = 1,4 Hz, b'-H), 7,82 (d, 1H, *J*= 1,0 Hz, b-H ou f-H), 7,84 (1H, *J*= 1,8 Hz, b-H ou f-H) ppm. $^{13}$C-RMN ($CDCl_3$): $\delta$ = 113,4 (C-6, C-3), 126,6, 127,5, 128,8, 129,3, 130,8, 130,9, 132,9, 147,8 (C-5), 152,9 (C-4), 169,0 (C-2) ppm. IR (KBr) : $\nu$ = 3100, 3063, 1753 (C=O), 1712, 1681, 1591, 1503, 1493, 1447, 1421, 1305, 1223, 1184, 1093, 978, 939, 922, 871, 830, 802, 767, 756, 690, 678, 650, 610, 552, 534 $cm^{-1}$. UV ($CH_2Cl_2$) : $\lambda_{max}$ ($\varepsilon$) = 236 (14 700), 339 nm (24 200 $Lmol^{-1}cm^{-1}$). SMRH (EI) : calculé pour $C_{15}H_{10}O_2S$ : 254,0401 ; trouvé : 254,0398 ; $C_{15}H_{10}O_2S$ (254,31): C 70,84, H 3,96 ; trouvé C 70,73, H 4,05.

- (Z)-4-bromo-5-(thiophène-3-ylméthylène)furane-2(*5H*)-one (6b) :

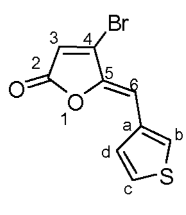

Dans un tube de Schlenk, sous atmosphère inerte, contenant 2 (200 mg, 0,78 mmol), l'acide thiophène-3-boronique (110 mg, 0,86 mmol), $K_3PO_4$ (334 mg, 1,57 mmol), S-Phos 10 (6 mg, 2 mol %) et $Pd(OAc)_2$ (27 mg, 3 mol %), est ajouté du toluène (3 mL). Le tube de Schlenk est ensuite bouché avec un septum, et le milieu réactionnel est agité pendant 5 h à température ambiante. Du $CH_2Cl_2$ (30 mL) est ajouté au milieu réactionnel qui est ensuite filtré sur un fritté contenant un peu de silice. Les solvants sont évaporés à l'évaporateur rotatif, et le résidu est chromatographié sur silice, avec comme éluant un mélange $CH_2Cl_2$/pentane (50/50). Un solide jaune est obtenu avec 27 % de rendement (54 mg, 0,21 mmol).
$R_f$ = 0,78 ($CH_2Cl_2$/pentane 50:50), p. f. 68-70°C. $^1$H-RMN ($CDCl_3$) : $\delta$ = 6,38 (s, 1H, 6-H), 6,45 (s, 1H, 3-H), 7,37 (dd,

1H, *J*= 5,1 Hz, *J*= 2,8 Hz, d-H), 7,56 (d, 1H, *J* = 5,1 Hz, c-H), 7,82 (d, 1H, *J*= 1,8 Hz, b-H) ppm. $^{13}$C-RMN ($CDCl_3$) : δ = 107,6 (C-6), 118,6 (C-3), 126,5, 128,7, 129,8, 133,6, 137,9 (C-5), 145,8 (C-4), 167,0 (C-2) ppm. IR (KBr) ν = 3122, 3062, 2924, 1752 (C=O), 1650, 1545, 1508, 1414, 1385, 1333, 1288, 1243, 1222, 1192, 1173, 1148, 1110, 1080, 976, 951, 939, 910, 881, 852, 835, 821, 795, 776, 709, 662, 629 $cm^{-1}$. UV ($CH_2Cl_2$) : $\lambda_{max}$ (ε) = 242 (10 700), 351 nm (31 900 $Lmol^{-1}cm^{-1}$). SMRH (EI) : calculé pour $C_9H_5O_2BrS$ : 255,9194 ; trouvé 255,9202 ; $C_9H_5O_2BrS$ (257,10) : C 42,04, H 1,96 ; trouvé C 42,16, H 2,04.

- 4-phényl-(Z)-5-(thiophène-3-ylméthylène)furane-2(*5H*)-one (8b) :

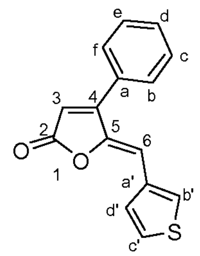

Dans un tube de Schlenk, sous atmosphère inerte, contenant 6b (101 mg, 0,39 mmol), l'acide phénylboronique (70 mg, 0,58 mmol), $K_3PO_4$ (123 mg, 0,58 mmol), S-Phos 10 (3 mg, 2 mol %) et $Pd(OAc)_2$ (8 mg, 3 mol %), est ajouté du toluène (3 mL). Le tube de Schlenk est ensuite bouché avec un septum, et le milieu réactionnel est agité pendant 24 h à 110°C. Après retour à température ambiante, du $CH_2Cl_2$ (30 mL) est ajouté au milieu réactionnel qui est ensuite filtré sur un fritté contenant un peu de silice. Les solvants sont évaporés à l'évaporateur rotatif et le résidu est chromatographié sur silice, avec comme éluant un mélange $CH_2Cl_2$/pentane (50/50). Un solide jaune est obtenu avec 66 % de rendement (66 mg, 0,26 mmol).

$R_f$ = 0,65 ($CH_2Cl_2$/pentane, 60:40), p. f. 70-72°C. $^1$H-RMN ($CDCl_3$) : δ = 6,19 (d, 1H, *J*= 0,6 Hz, 3-H), 6,28 (s, 1H, 6-H), 7,35 (dd, 1H, *J*= 5,1 Hz, *J*= 3,0 Hz, d'-H), 7,48-7,57 (m, 6H, c'-H, b-H, c-H, d-H, e-H, f-H), 7,77 (dd, 1H, *J* = 2,9 Hz, *J*= 0,6 Hz, b'-H) ppm. $^{13}$C-RMN ($CDCl_3$) : δ = 107,9 (C-6), 114,3 (C-3), 126,2, 128,4, 128,7, 129,0, 129,1, 130,4, 134,4, 147,0 (C-5), 158,3 (C-4), 168,6 (C-2) ppm. IR (KBr) : ν = 3101, 1759 (C=O), 1644, 1570, 1514, 1489, 1414, 1348, 1248, 1196, 1085, 937, 917, 875, 837, 812, 771, 703, 665, 636 $cm^{-1}$. UV ($CH_2Cl_2$) : $\lambda_{max}$ (ε) = 228 (11 400), 351 nm (17 000 $Lmol^{-1}cm^{-1}$). SMRH (EI): calculé pour $C_{15}H_{10}O_2S$ : 254,0401 ; trouvé 254,0374 ; $C_{15}H_{10}O_2S$ (254,04) : C 70,84, H 3,96 ; trouvé C 70,68, H 4,03.

- (Z)-5-(bromophénylméthylene)furan-2(5*H*)-one (7a) :

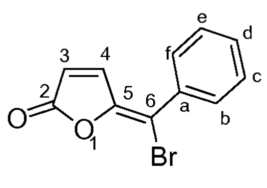

Dans un tube de Schlenk, sous atmosphère inerte, contenant 3 (180 mg, 0,71 mmol), l'acide phénylboronique (129 mg, 1,06 mmol), $Na_2CO_3$ (150 mg, 1,42 mmol) et $Pd(PPh_3)_4$ (24 mg, 3 mol %) sont ajoutés du toluène (3 mL), $H_2O$ (1,5 mL) et de l'éthanol (0,9 mL). Le tube de Schlenk est ensuite bouché avec un septum, et le milieu réactionnel est agité pendant 24 h à température ambiante. De l'eau (30 mL) est ensuite ajoutée au milieu réactionnel. Il est extrait avec de l'éther (3 ×20 mL). Les phases organiques réunies sont lavées avec une solution d'eau saturée en NaCl et séchées sur $Na_2SO_4$. Les solvants sont évaporés à l'évaporateur rotatif, et le résidu est chromatographié sur silice avec comme éluant un mélange $Et_2O$/pentane (25/75). Un solide blanc crème est obtenu avec 48 % de rendement (94 mg, 0,37 mmol).

$R_f$ = 0,45 ($Et_2O$/pentane, 75:25), p. f. < 50°C. $^1$H-RMN ($CDCl_3$) : δ = 6,32 (d, 1H, *J* = 5,4 Hz, 3-H), 7,43-7,46 (m, 6H, $H_4$, ArH) ppm. $^{13}$C-RMN ($CDCl_3$) : δ = 110,9 (C-6), 120,8 (C-3), 128,8, 130,1, 130,2, 135,4, 140,9 (C-4), 149,1 (C-5), 168,5 (C-2) ppm. IR (KBr) : ν = *3135,* 3096, 3031, 1788 (C=O), 1774 (C=O), 1628, 1612, 1548, 1540, 1486, 1443, 1230, 1106,

1072, 956, 920, 901, 863, 860, 817, 806, 749, 711, 689, 625, 535 $cm^{-1}$. UV ($CH_2Cl_2$) : $\lambda_{max}$ ($\varepsilon$) = 321 nm (20 400 $Lmol^{-1}cm^{-1}$). SMRH (EI): calculé pour $BrC_{11}H_7O_2$ : 249,9630; trouvé 249,9628; $BrC_{11}H_7O_2$ (251,08) : C 52,62, H 2,81 ; trouvé C 52,61, H, 2,90.

- (Z)-5-(phénylthiophène-3-ylméthylène)furane-2(5H)-one (9a) :

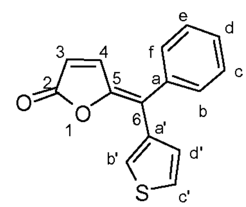

Dans un tube de Schlenk, sous atmosphère inerte, contenant 7a (480 mg, 1,91 mmol), l'acide thiophène-3-boronique (366 mg, 2,86 mmol), $Na_2CO_3$ (404 mg, 3,82 mmol), $Pd(PPh_3)_4$ (66 mg, 3 mol %) sont ajoutés du toluène (10 mL), $H_2O$ (5 mL) et de l'éthanol (2,5 mL). Le tube de Schlenk est ensuite bouché avec un septum, et le milieu réactionnel porté à 80°C pendant 2,5 h. Après retour à température ambiante, de l'eau (50 mL) est ajoutée au milieu réactionnel. Il est extrait avec de l'éther (3 $\times$ 20 mL). Les phases organiques réunies sont lavées avec 50 mL d'une solution d'eau saturée en NaCl et séchées sur $Na_2SO_4$. Les solvants sont évaporés à l'évaporateur rotatif et le résidu est chromatographié sur silice avec comme éluant un mélange $CH_2Cl_2$/pentane (70/30).Un solide jaune pâle est obtenu avec 48 % de rendement (233 mg, 0,92 mmol).
$R_f$ = 0,50 ($CH_2Cl_2$/pentane, 70:30). $^1H$-RMN ($CDCl_3$) : $\delta$ = 5,71 (d, 1H, *J* = 5,4 Hz, 3-H), 7,28-7,37 (m, 3H, b'-H, c'-H, d'-H), 7,40-7,52 (m, 5H, 4-H, b-H, c-H, d-H, eH, f-H) ppm. $^{13}C$-RMN ($CDCl_3$) : $\delta$ = 117,8 (C-3), 123,1 (C-6), 125,5, 128,4, 128,9, 129,5, 129,9, 130,8, 136,5, 137,7, 143,9 (C-4), 146,2 (C-5), 170,2 (C-2) ppm. IR : $\nu$ = 3133, 3106, 3057, 2926, 1777 (C=O), 1752 (C=O), 1607, 1544, 1504, 1442, 1410, 1372, 1337, 1301, 1235, 1216, 1166, 1108, 1068, 1000, 979, 954, 892, 849, 807, 746, 712, 699, 682 $cm^{-1}$. UV ($CH_2Cl_2$) : $\lambda_{max}$ ($\varepsilon$) = 254 (9 000), 354 nm (19 000 $Lmol^{-1}cm^{-1}$). SMRH (EI) : calculé pour $C_{15}H_{10}O_2S$: 254,0401 ; trouvé 254,0398 ; $C_{15}H_{10}O_2S$ (254,30) : C 70,84, H 3,96 ; trouvé C 70,66, H 3,91.

- (Z)-5-(bromothiophène-3-ylméthylène)furane-2(5*H*)one (7b) :

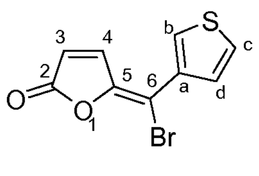

Dans un tube de Schlenk, sous atmosphère inerte, contenant 2 (245 mg, 0,96 mmol), l'acide thiophène-3-boronique (185 mg, 1,44 mmol), $Na_2CO_3$ (204 mg, 1,93 mmol), $Pd(PPh_3)_4$ (33 mg, 3 mol %), sont ajoutés du toluène (5 mL), $H_2O$ (2,45 mL) et de l'éthanol (1,22 mL). Le tube de Schlenk est ensuite bouché avec un septum, et le milieu réactionnel est agité pendant 3 jours à température ambiante. De l'eau (30 mL) est ensuite ajoutée au milieu réactionnel. Il est extrait avec de l'éther (3 $\times$ 20 mL). Les phases organiques réunies sont lavées avec 30 mL d'une solution d'eau saturée en NaCl et séchées sur $Na_2SO_4$. Les solvants sont évaporés à l'évaporateur rotatif et le résidu est chromatographié sur silice avec comme éluant un mélange $Et_2O$/pentane (35/65). Un solide jaune pâle est obtenu avec 40 % de rendement (99 mg, 0,38 mmol).
$R_f$ = 0,65 ($Et_2O$/pentane, 75:25), p. f. 65-67°C. $^1H$-RMN ($CDCl_3$) : $\delta$ = 6,33 (d, 1H, *J* = 5,6 Hz, 3-H), 7,25 (dd, 1H, *J*= 6,4 Hz, *J*= 1,3 Hz, d-H), 7,43 (dd, 1H, *J*= 4,9 Hz, *J*= 3,1 Hz, c-H), 7,47 (dd, 1H, *J* = 2,9 Hz, *J*= 1,4 Hz, b-H), 7,59 (d, 1H, *J*= 5,4 Hz, 4-H) ppm. $^{13}C$-RMN ($CDCl_3$) : $\delta$ = 105,2 (C-6), 120,6 (C-3), 127,1, 127,4, 128,6, 136,0, 140,6 (C-4), 148,9 (C-5), 168,4 (C-2) ppm. IR (KBr) : $\nu$ = 3135, 3107, 2916, 2848, 1781 (C=O), 1756 (C=O), 1621, 1546, 1512, 1410, 1366, 1335, 1261, 1230, 1181, 1108, 1070, 991, 928, 890, 839, 807, 781, 748, 696 $cm^{-1}$. UV ($CH_2Cl_2$) : $\lambda_{max}$ ($\varepsilon$) = 255 (9 100), 334 nm (20 400 $Lmol^{-1}cm^{-1}$). SMRH (EI): calculé pour $BrC_9H_5O_2S$ : 255,9194 ; trouvé 255,9202 ; $BrC_9H_5O_2S$ (257,10) : C 42,04, H 1,96 ; trouvé C 42,12, H 2,02.

- (E)-5-(phénylthiophène-3-ylméthylène)furane-2(5*H*)-one (9b) :

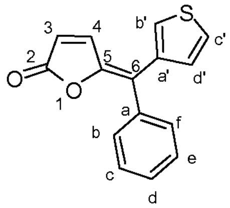

Dans un tube de Schlenk, sous atmosphère inerte, contenant **7b** (76 mg, 0,29 mmol), l'acide phénylboronique (54 mg, 0,44 mmol), $Na_2CO_3$ (62 mg, 0,59 mmol) et $Pd(PPh_3)_4$ (13 mg, 3 mol %), sont ajoutés du toluène (2 mL), $H_2O$ (1 mL) et de l'éthanol (0,5 mL). Le tube de Schlenk est ensuite bouché avec un septum, et le milieu réactionnel porté à 80°C pendant 5 h. Après retour à température ambiante, de l'eau (5 mL) est ajoutée au milieu réactionnel. Il est extrait avec de l'éther (3 × 15 mL). Les phases organiques réunies sont lavées avec une solution d'eau saturée en NaCl et séchées sur $Na_2SO_4$. Les solvants sont évaporés à l'évaporateur rotatif et le résidu est chromatographié sur silice avec comme éluant un mélange $Et_2O$/pentane (30/70). Une huile jaune est obtenue avec 42 % de rendement (31 mg, 0,12 mmol).
$R_f$ = 0,39 ($Et_2O$/pentane, 25:75), p. f. 118-119°C. $^1$H-RMN ($CDCl_3$) : $\delta$ = 6,22 (d, 1H, *J* = 5,6 Hz, H-3), 6,98 (dd, 1H, *J* = 4,9 Hz, *J* = 1,3 Hz, d'-H), 7,31 (dd, 1H, *J* = 3,1 Hz, *J* = 1,3 Hz, b'-H), 7,35-7,42 (m, 4H, c'-H et b-H ou c-H ou d-H ou e-H ou f-H), 7,49-7,53 (m, 2H, b-H ou c-H ou d-H ou e-H ou f-H), 7,62 (d, 1H, *J* = 5,6 Hz, 4-H) ppm. $^{13}$C-RMN ($CDCl_3$) : $\delta$ = 118,5 (C-3), 123,6 (C-6), 126,3, 127,2, 128,2, 129,2, 129,8, 130,9, 136,1, 137,6, 143,5 (C-4), 147,2 (C-5), 170,4 (C-2) ppm. IR 3132, 3102, 3049, 1771 (C=O), 1755 (C=O), 1617, 1573, 1540, 1489, 1444, 1416, 1233, 1166, 1103, 1084, 990, 897, 874, 850, 816, 792, 750, 694, 671, 656, 613 $cm^{-1}$. UV ($CH_2Cl_2$) : $\lambda_{max}$ ($\varepsilon$) = 250 (10 900), 352 nm (21 400 $Lmol^{-1}cm^{-1}$). SMRH (EI) : calculé pour $C_{15}H_{10}O_2S$ : 254,0401 ; trouvé 254,0398 ; $C_{15}H_{10}O_2S$ (254,31): C 70,84, H 3,96 ; trouvé C 70,71, H 3,99.

- 5-(9H-fluoren-9-ylidene)furan-2(5H)-one (11)

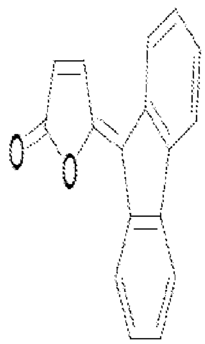

(11)

Rf (EtOAc/cyclohexane 1:2) = 0.44
HRMS calc. $M^+$ 246.0681, trouvée 246.0667.
$\delta_H$ (270 MHz, $CDCl_3$): 8.36-8.34 (2H, m, H aromatique), 7.73-7.68 (3H, m, 2H aromatique and 1H olefinique), 7.39-7.25 (4H, m, H aromatique), 6.48 (1H, d, *J* = 5.38 Hz, 1H olefinique).
$\delta_C$ (67.80 MHz, $CDCl_3$): 170.65, 147.60, 141.80, 140.80, 140.55, 135.85, 135.44, 129.93, 129.39, 128.38, 128.10, 127.36, 123.70, 120.79, 120.58, 119.79.

Les présents inventeurs ont démontré que l'ensemble des dérivés de méthylène furanone de formule générale (I), tels que revendiqués dans la présente invention, possèdent une activité photoprotectrice et antioxydante.

Il est connu que la peau constitue une cible directe de nombreux agents environnementaux dont les rayons ultraviolets de types A et B, les UVC étant arrêtés par la couche d'ozone.

Les UVB (5 % de l'énergie UV terrestre) atteignent l'épiderme cutané tandis que les UVA (95 % de l'énergie

terrestre) sont plus pénétrants puisqu'ils atteignent le derme cutané. Les conséquences biologiques de ce stress UV sont décrites comme une augmentation de la réponse inflammatoire et le développement d'un érythème, une modulation de la réponse immune et, à plus long terme, un vieillissement prématuré et la promotion de cancers cutanés.

Les UVA et UVB, par des voies d'accès différentes, perturbent la stabilité génomique. Les UVB sont principalement absorbés par la molécule d'ADN et génèrent majoritairement des lésions encombrantes comme les dimères de pyrimidine de type cyclobutane et de type 6-4 pyrimidine-pyrimidone. Ces photoproduits sont réparés spécifiquement par des systèmes de défense cellulaires, tels que la réparation par excision de nucléotides.

Les UVA sont absorbés principalement par d'autres molécules que l'ADN, dites chromophores, générant ainsi la production de radicaux libres oxygénés capables d'attaquer l'ADN et créant de nombreuses lésions, telles que des cassures de brins, directes ou dues aux sites alcali labiles. La cellule possède également des systèmes de réparation spécifiques de ces lésions sur l'ADN.

Aujourd'hui, il est clairement admis que les rayonnements UVA et UVB peuvent être mutagènes et constituer des promoteurs potentiels de la photocarcinogénèse cutanée. Il semble donc impératif de protéger la peau des effets délétères des ultraviolets par des écrans solaires topiques possédant des filtres puissants contre les UVA et/ou les UVB.

Les dérivés de méthylène furanone de formule générale (I) de la présente invention ont ainsi été évalués afin de déterminer leur pouvoir photoprotecteur par détermination de leurs caractéristiques d'absorption UV : coefficient d'extinction molaire $\varepsilon$, longueur d'onde critique ($\lambda c$) et rapport UVA/UVB.

Les résultats obtenus montrent que les coefficients d'extinction molaire de nos composés s'échelonnent de 12 300 $L.mol^{-1}.cm^{-1}$ à 31 900 $L.mol^{-1}.cm^{-1}$ dont une large majorité au-dessus de 20 000 $L.mol^{-1}.cm^{-1}$, ce qui est tout à fait dans la gamme des produits utilisés dans l'industrie cosmétique. Mis à part **4b** et **4c** dont le maximum est en UVB, ils possèdent tous un maximum d'absorption en UVA. La longueur d'onde critique confirme cette observation ; effectivement celle-ci est supérieure à 370 nm pour la majorité des molécules. Le ratio UVA/UVB nous indique que nous pourrions être en présence de filtres larges ($R \geq 1,5$), qui couvrent bien les UVA mais également une partie des UVB.

En regroupant toutes ces informations et en comparant l'allure des spectres d'absorption UV, nous pouvons classer ces molécules en trois catégories :

- Catégorie UVA stricte (voir figure 1 en annexe)
  (molécules absorbant uniquement en UVA) :
  5a, 4c, 6b, 9b, 9a, 5b, 4d, 5e, 5d

$$\lambda c > 370 \text{ nm}$$

$$3 < r < 6,5$$

  Dans cette catégorie seuls **5a** et **6b** possèdent un $\lambda c < 370$ (respectivement 367 et 355 nm). Toutes ces molécules absorbent dans les UVA longs (340-400 nm) ce qui est intéressant car peu de filtres commerciaux, actuellement, couvrent cette zone. On peut également remarquer que les composés substitués par des groupements donneurs absorbent en général à plus haute longueur d'onde.

- Catégorie UVA étendue vers B (voir figure 2 en annexe)
  (molécules absorbant majoritairement
  en UVA et minoritairement en UVB) :
  7a, 5f, 7b, 4a, 8a, 6a, 4f, 8b, 5c

$$\lambda c < 370 \text{ nm}$$

$$1,5 < r < 3$$

  Comme indiqué sur le spectre d'absorption ci-dessus en exemple, ces molécules possèdent un maximum d'absorption en UVA mais couvrent également une partie importante des UVB. Ceci est confirmé par la valeur du rapport UVA/UVB, qui est comprise entre 1,5 et 3 pour 8 de ces 9 composés. Une majorité de ces molécules absorbe également dans les UVA longs (4a, 8a, 6a, 4f, 8b, 5c).

- Catégorie UVA + B (voir figure 3 en annexe)
  (molécules ayant un maximum d'absorption en UVA et en UVB) :

4b, 4c

$$\lambda c > 370\ \text{nm}$$

$$r = 1{,}9\ \text{et}\ 3{,}2$$

Ces deux molécules possèdent également des propriétés intéressantes, puisqu'elles absorbent en UVA et en UVB, et plus particulièrement **4c** avec un bon coefficient d'extinction molaire de 22 000 L.mol$^{-1}$.cm$^{-1}$.

Photostabilité

Des tests de photostabilité ont été effectués sur sept molécules à savoir (4d, 5a, 5b, 5d, 5e et 9a (UVA strict) ainsi que 5f (UVA étendu vers B):

**4d** **5a** **5b** **5d**

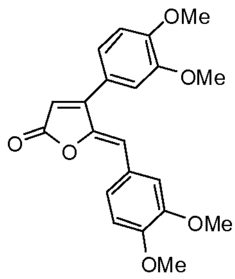
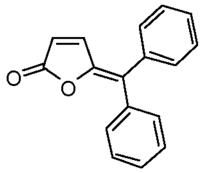
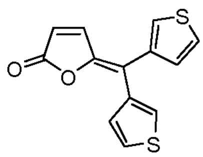
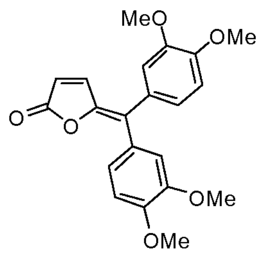

**5e** **9a** **5f**

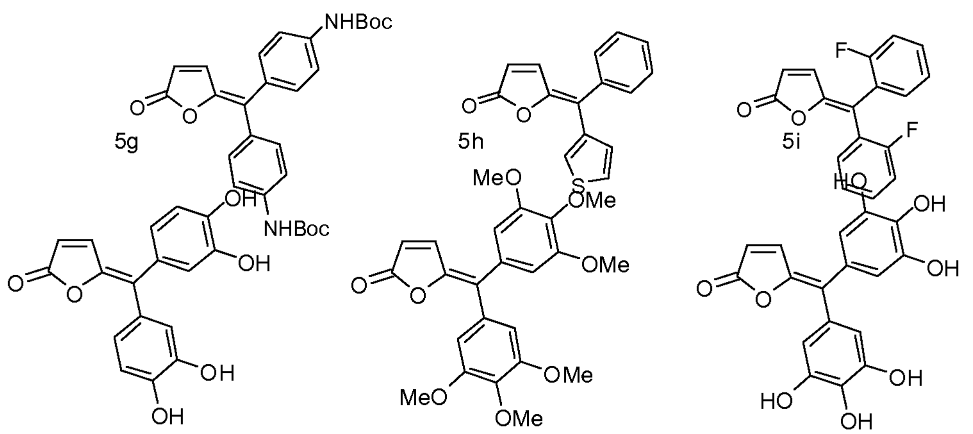

5g 5h 5i

Pour cette étude il a été choisi d'irradier les échantillons, sous forme de poudre ou en solution dans l'éthanol, avec 10 DEM (Dose Erythémale Minimale : dose d'ensoleillement minimale provoquant l'apparition d'un coup de soleil). L'évaluation de la dégradation est estimée par l'allure des spectres d'absorption UV avant et après irradiation.

| Molécules | En solution | En poudre |
|---|---|---|
| 4d | moyennement stable | stable |
| 5a | stable | stable |
| 5b | stable | stable |
| 5d | stable | pas stable |
| 5e | stable | stable |
| 9a | stable | stable |

(suite)

| Molécules | En solution | En poudre |
|---|---|---|
| 5f | stable | stable |

*Evaluation de la stabilité des dérivés de méthylène furanone irradiés au Suntest*

Les résultats rassemblés dans le tableau ci-dessus indiquent que cinq des sept composés testés présentent une très bonne photostabilité. Seul **4d** est moyennement stable en solution, et **5d** est totalement instable sous forme de poudre.

**Etude du pouvoir antioxydant des molécules**

La mesure du pouvoir antioxydant de 10 molécules a été réalisée par piégeage de l'anion superoxyde : 5d, 9a, 5b, 5a, 5e, 4d, 5f, 5g, 5h, 5j.
La méthode consiste à générer des radicaux libres par un signal photochimique. L'intensité de l'oxydation est 1000 fois supérieure à celles obtenues en conditions normales.
Ainsi le radical superoxyde : $\mathbf{O_2^-}$ est généré par une réaction photochimique :

$$L + hv\ (UV) + O_2 \rightarrow L^*\mathbf{O_2} \rightarrow \mathbf{L + O_2{-}}$$

*L* : luminol à l'état excité*
*$L^+$ : radical luminol*

La détection se fait par chimioluminescence. Elle permet l'évaluation de molécules ou extraits antioxydants hydrosolubles et liposolubles.
Une partie des anions superoxides est quenchée par les antioxydants. Les radicaux libres restants sont quantifiés par chimioluminescence.

$$L^+ + \mathbf{O_2^-} \rightarrow N2 + AP^{*2-} \rightarrow AP^{2-} + hv\ (luminescence)$$

$AP^{*2-}$ : *aminophtalate à l'état excité*
Les résultats sont exprimés respectivement en quantité équivalente de vitamine C ou de Trolox (acide 6-hydroxy-2,5,7,8-tétraméthylchroman-2-carboxylique). La sensibilité est de l'ordre de la nanomole.

| Nom | Conditions | Photosensibilisant | Anti-oxydant |
|---|---|---|---|
| Blanc | 100% $O_2^-$ générés | + | - |
| Standards | Gamme standard: de 1 à 3 nmol | + | Vitamine C ou Trolox |
| Essai | +/- $O_2^-$ générés | + | Molécule x |

L'analyse des résultats dépend de deux critères : de l'allure de la courbe et de la valeur numérique donnée par le logiciel.
Les résultats seront exprimés en µg d'échantillon nécessaire pour obtenir une activité équivalente à l'activité détectée pour 1 µg de standard (acide ascorbique ou Trolox).

| Produits | µg d'échantillon pour1 µg de Trolox |
|---|---|
| Vitamine C | 0,451 |
| Trolox | 1,067 |
| Butylhydroxyanisole (BHA) | 2,650 |
| Bilirubine | 4,18 |
| Butylhydroxytoluène (BHT) | 7,26 |
| Idébénone | 10,10 |

(suite)

| Produits | μg d'échantillon pour1 μg de Trolox |
|---|---|
| α-tocophérol-acétate | 140 |
| Cystéine | 698 |
| Albumine | 3069 |
| Acide Lipoïque | NEGATIF |

| Echantillon | ACL |
|---|---|
| | μg d'échantillon pour 1 μg de Trolox |
| 5d | 12,7 |
| 9a | 21,6 |
| 5b | 28,0 |
| 5a | 43,0 |
| 5e | 46,3 |
| 4d | 50,2 |
| 5f | 88,3 |
| 5g | 0,03 |
| 5h | 30 |
| 5i | 1,5 |

- Etude du pouvoir dépigmentant des composés méthylène furanone (poly)hydroxylés:

*- Cible :*

La tyrosinase est une enzyme limitante dans la mélanogénèse.

Elle appartient à la famille des oxydoréductases. Elle possède notamment la fonction : monophenol mono oxygenase et polyphenol oxydase.

Elle est synthétisée au niveau des mélanocytes. Elle est activée lors de sa migration vers les kératinocytes via les mélanosomes. Elle transforme la tyrosine en DOPA puis dopaquinone, ce qui conduit *in fine* à une polymérisation soit une production de pigments

*-principe:*

C'est la fonction monophenol monooxygenase qui est mesurée dans ce test. Nous mesurons une activité tyrosinase : MPMO *stricto sensus.*

Le substrat : la L-tyrosine est transformée en L-DOPA. La réaction enzymatique est stoppée à ce stade. La DOPA produite est déterminée par une calibration externe (0,005 à 0,1 mM). Le résultat final est un pourcentage d'inhibition par rapport à une activité maximum sans inhibiteur ou extrait.

*-Résultat:*

Pour une quantité de 50 μM, le composé 5g inhibe de 65, 93% l'activité de l'enzyme MPMO.

Conclusion

Les tests basés sur les **données** d'absorption UV ont montré que ces nouveaux dérivés de méthylène furanone absorbent principalement en UVA avec de bons coefficients d'extinction molaire. L'allure des spectres a permis, de les classer en trois catégories : UVA strict, UVA étendu vers B, et UVA + B. Les méthylène furanones gem-disusbtitués

sont majoritairement présentes dans la catégorie UVA strict. L'introduction d'aromatiques portant des groupes méthoxy ou aminocarbamate attracteurs semblent responsable d'un effet bathochrome car certaines des molécules absorbent dans les UVA longs vers 380 nm.

Les tests réalisés sur ces lactones indiquent que la plupart de ces molécules sont photostables et que ce sont également de bons antioxydants. Ces composés présentent donc des capacités photoprotectrices et antioxydantes intéressantes qui justifient leur utilisation dans des compositions dermo-cosmétiques.

Le test d'inhibition de l'enzyme MPMO a permis de démontrer les propriétés dépigmentantes des composés méthylène furanone (poly)hydroxylés.

## Revendications

**1.** Dérivés de méthylène furanone de formule générale (I) :

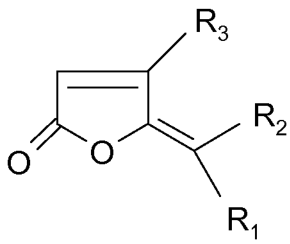

(I)

sous leurs formes isomères (E) ou (Z), pures ou en mélange, dans laquelle

- R3 représente un hydrogène

- $R_1$ et $R_2$ sont identiques et représentent

- un radical phényle substitué par un ou plusieurs radicaux

◦ alkyl($C_{1-5}$), alkyl($C_{1-4}$)oxy,
◦ hydroxy,
◦ méthylthio,
◦ halogéno, ou
◦ un groupement amino éventuellement substitué par un radical alkyl($C_{1-4}$), alkyl($C_{1-4}$)oxycarbonyle,

- un radical thiophényle,
- un radical thionaphtènyle,
- un radical thiazolyle,
- un radical pyridinyle,
- un radical benzomorpholinyle,
- un radical naphtyle

et
à la condition toutefois que $R_1$ et $R_2$ ne représentent pas simultanément un radical paraméthoxyphényle.

**2.** Dérivés de méthylène furanone de formule générale (I) selon la revendication (1) **caractérisés en ce que** :

- R3 représente un hydrogène
- R1 et R2 sont identiques et représentent

- un radical phényle substitué par un ou plusieurs radicaux

- alkyl($C_{1-4}$)oxy,
- hydroxy,

- un radical thiophényle,

et à la condition toutefois que $R_1$ et $R_2$ ne représentent pas simultanément un radical paraméthoxyphényle.

3. Dérivés de méthylène furanone de formule générale (I) selon l'une des revendications 1 à 2 **caractérisés en ce que** :

- R3 représente un hydrogène
- R1 et R2 sont identiques et représentent
    - un radical phényle substitué par un ou plusieurs radicaux
        - hydroxy

4. Dérivés de méthylène furanone de formule générale (I) selon l'une des revendications 1 à 3, sous leurs formes isomères (E) ou (Z), pures ou en mélange, choisis parmi :

- 5-(bis(3,4-diméthoxyphényl)méthylène)furane-2(5*H*)-one ;
- 5-(dithiophène-3-ylméthylène)furane-2(5*H*)-one ;
- 5-bis(4-hydroxyphénylcarbamate de *tert*-butyl)furane-2(5*H*)-one ;
- 5-(bis(2-fluorophényl)méthylène)furane-2(5*H*)-one ;
- 5-(bis(4-(méthylthio)phényl)méthylène)furan-2(5H)-one
- 5-(bis(4-(diméthylamino)phényl)méthylène)furan-2(5H)-one
- 5-(bis(4-méthyl-3,4-dihydro-2H-benzo[b][1,4]oxazin-7-yl)méthylène)furan-2(5H)-one
- 5-(dithiazol-2-ylméthylène)furan-2(5H)-one
- 5-(bis(4-fluorophényl)méthylène)furan-2(5H)-one
- 5-(bis(4-hydroxy-3,5-diméthylphényl)méthylène)furan-2(5H)-one
- 5-(bis(3,4,5-trihydroxyphényl)méthylène)furan-2(5H)-one
- 5-(bis(3,4-dihydroxyphényl)méthylène)furan-2(5H)-one
- 5-(bis(4-hydroxy-3-méthoxyphényl)méthylène)furan-2(5H)-one
- 5-(bis(4-hydroxy-3,5-diméthoxyphényl)méthylène)furan-2(5H)-one
- 5-(bis(4-hydroxyphényl)méthylène)furan-2(5H)-one
- 5-(bis(2,4-dimethoxyphenyl)methylene)furan-2(5H)-one
- 5-(bis(2,4-dihydroxyphenyl)methylene)furan-2(5H)-one

5. Utilisation cosmétique de dérivés de méthylène furanone de formule générale (I):

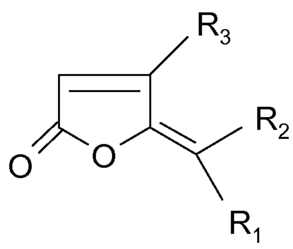

(I)

sous leurs formes isomères (E) ou (Z), pures ou en mélange, dans laquelle l'un des radicaux $R_1$ et $R_3$ représente nécessairement un hydrogène, et $R_1$, $R_2$, $R_3$ sont identiques ou différents et représentent :

- un radical phényle éventuellement substitué par un ou plusieurs radicaux
    - ◦ alkyl($C_{1-5}$ ), alkyl($C_{1-4}$)oxy,
    - ◦ hydroxy,
    - ◦ méthylthio,
    - ◦ halogéno, ou

◦ un groupement amino éventuellement substitué par un radical alkyl($C_{1-4}$), alkyl($C_{1-4}$)oxycarbonyle,

- un radical thiophényle,
- un radical thionaphtènyle,
- un radical thiazolyle,
- un radical pyridinyle,
- un radical benzomorpholinyle,
- un radical naphtyle,
- R1 et R2 sont reliés entre eux et représentent un radical fluorenyl

en tant qu'agent photoprotecteur.

**6.** Utilisation cosmétique selon la revendication 5, **caractérisée en ce que** les dérivés de méthylène furanone de formule générale (I), sont sous leurs formes isomères (E) ou (Z), pures ou en mélange,
et **caractérisée en ce que**

- $R_3$ représente un hydrogène, et
- $R_1$ et $R_2$ sont identiques ou différents et représentent

  - un radical phényle éventuellement substitué par un ou plusieurs radicaux

    ◦ alkyl($C_{1-5}$ ), alkyl($C_{1-4}$)oxy,
    ◦ hydroxy,
    ◦ méthylthio,
    ◦ halogéno, ou
    ◦ un groupement amino éventuellement substitué par un radical alkyl($C_{1-4}$), alkyl($C_{1-4}$)oxycarbonyle,

  - un radical thiophényle,
  - un radical thionaphtènyle,
  - un radical thiazolyle,
  - un radical pyridinyle,
  - un radical benzomorpholinyle,
  - un radical naphtyle,
  - R1 et R2 sont reliés entre eux et représentent un radical fluorenyl.

**7.** Dérivés de méthylène furanone de formule générale (I):

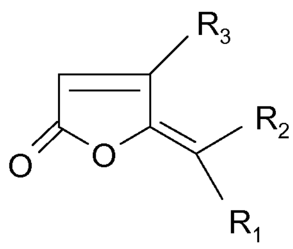

(I)

sous leurs formes isomères (E) ou (Z), pures ou en mélange,
dans laquelle
l'un des radicaux $R_1$ et $R_3$ représente nécessairement un hydrogène,
et $R_1$, $R_2$, $R_3$ sont identiques ou différents et représentent :

- un radical phényle éventuellement substitué par un ou plusieurs radicaux

  ◦ alkyl($C_{1-5}$ ), alkyl($C_{1-4}$)oxy,
  ◦ hydroxy,
  ◦ méthylthio,
  ◦ halogéno, ou

- ◦ un groupement amino éventuellement substitué par un radical alkyl($C_{1-4}$), alkyl($C_{1-4}$)oxycarbonyle,

- un radical thiophényle,
- un radical thionaphtènyle,
- un radical thiazolyle,
- un radical pyridinyle,
- un radical benzomorpholinyle,
- un radical naphtyle,
- R1 et R2 sont reliés entre eux et représentent un radical fluorenyl

pour utilisation dermatologique en tant qu'agent photoprotecteur.

**8.** Dérivés pour utilisation selon la revendication 7, **caractérisé en ce que** les dérivés de méthylène furanone de formule générale (I), sont sous leurs formes isomères (E) ou (Z), pures ou en mélange,
et **caractérisé en ce que**

- $R_3$ représente un hydrogène, et
- $R_1$ et $R_2$ sont identiques ou différents et représentent

  - un radical phényle éventuellement substitué par un ou plusieurs radicaux

    - ◦ alkyl($C_{1-5}$ ), alkyl($C_{1-4}$)oxy,
    - ◦ hydroxy,
    - ◦ méthylthio,
    - ◦ halogéno, ou
    - ◦ un groupement amino éventuellement substitué par un radical alkyl($C_{1-4}$), alkyl($C_{1-4}$)oxycarbonyle,

  - un radical thiophényle,
  - un radical thionaphtènyle,
  - un radical thiazolyle,
  - un radical pyridinyle,
  - un radical benzomorpholinyle,
  - un radical naphtyle,

R1 et R2 sont reliés entre eux et représentent un radical fluorenyl.

**9.** Utilisation cosmétique de dérivés de méthylène furanone de formule générale (I) :

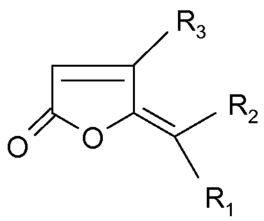

(I)

sous leurs formes isomères (E) ou (Z), pures ou en mélange,
dans laquelle
l'un des radicaux $R_1$ et $R_3$ représente nécessairement un hydrogène et $R_1$, $R_2$, $R_3$ sont identiques ou différents et représentent :

- un radical phényle éventuellement substitué par un ou plusieurs radicaux

  - ◦ alkyl($C_{1-5}$ ), alkyl($C_{1-4}$)oxy,
  - ◦ hydroxy,
  - ◦ méthylthio,
  - ◦ halogéno, ou

◦ un groupement amino éventuellement substitué par un radical alkyl($C_{1-4}$), alkyl($C_{1-4}$)oxycarbonyle,
- un radical thiophényle,

- un radical thionaphtènyle,
- un radical thiazolyle,
- un radical pyridinyle,
- un radical benzomorpholinyle,
- un radical naphtyle,
- R1 et R2 sont reliés entre eux et représentent un radical fluorenyl

en tant qu'agent antioxydant.

**10.** Utilisation cosmétique selon la revendication 9, **caractérisée en ce que** les dérivés de méthylène furanone de formule générale (I) sont sous leurs formes isomères (E) ou (Z), pures ou en mélange,
et **caractérisée en ce que**

- $R_3$ représente un hydrogène, et
- $R_1$ et $R_2$ sont identiques ou différents et représentent

  - un radical phényle éventuellement substitué par un ou plusieurs radicaux

    ◦ alkyl($C_{1-5}$ ), alkyl($C_{1-4}$)oxy,
    ◦ hydroxy,
    ◦ méthylthio,
    ◦ halogéno, ou
    ◦ un groupement amino éventuellement substitué par un radical alkyl($C_{1-4}$), alkyl($C_{1-4}$)oxycarbonyle,

  - un radical thiophényle,
  - un radical thionaphtènyle,
  - un radical thiazolyle,
  - un radical pyridinyle,
  - un radical benzomorpholinyle,
  - un radical naphtyle,
  - R1 et R2 sont reliés entre eux et représentent un radical fluorenyl.

**11.** Dérivés de méthylène furanone de formule générale (I) :

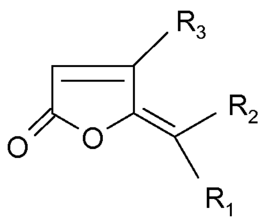

(I)

sous leurs formes isomères (E) ou (Z), pures ou en mélange,
dans laquelle
l'un des radicaux $R_1$ et $R_3$ représente nécessairement un hydrogène et $R_1$, $R_2$, $R_3$ sont identiques ou différents et représentent :

- un radical phényle éventuellement substitué par un ou plusieurs radicaux

  ◦ alkyl($C_{1-5}$ ), alkyl($C_{1-4}$)oxy,
  ◦ hydroxy,
  ◦ méthylthio,
  ◦ halogéno, ou
  ◦ un groupement amino éventuellement substitué par un radical alkyl($C_{1-4}$), alkyl($C_{1-4}$)oxycarbonyle,

- un radical thiophényle,
- un radical thionaphtènyle,
- un radical thiazolyle,
- un radical pyridinyle,
- un radical benzomorpholinyle,
- un radical naphtyle,
- R1 et R2 sont reliés entre eux et représentent un radical fluorenyl

pour utilisation dermatologique en tant qu'agent antioxydant.

**12.** Dérivés pour utilisation selon la revendication 11, **caractérisée en ce que** les dérivés de méthylène furanone de formule générale (I) sont sous leurs formes isomères (E) ou (Z), pures ou en mélange,
et **caractérisée en ce que**

- $R_3$ représente un hydrogène, et
- $R_1$ et $R_2$ sont identiques ou différents et représentent
  - un radical phényle éventuellement substitué par un ou plusieurs radicaux
    - ◦ alkyl($C_{1-5}$ ), alkyl($C_{1-4}$)oxy,
    - ◦ hydroxy,
    - ◦ méthylthio,
    - ◦ halogéno, ou
    - ◦ un groupement amino éventuellement substitué par un radical alkyl($C_{1-4}$), alkyl($C_{1-4}$)oxycarbonyle,
  - un radical thiophényle,
  - un radical thionaphtènyle,
  - un radical thiazolyle,
  - un radical pyridinyle,
  - un radical benzomorpholinyle,
  - un radical naphtyle,

R1 et R2 sont reliés entre eux et représentent un radical fluorenyl

**13.** Utilisation de dérivés de méthylène furanone de formule générale (I) selon la revendication 3 en tant qu'agent dépigmentant.

**14.** Composition dermocosmétique comprenant un excipient cosmétiquement acceptable et un dérivé de méthylène furanone selon l'une quelconque des revendications 1 à 4 en tant qu'agent photoprotecteur ou agent antioxydant.

**15.** Composition dermocosmétique comprenant un excipient cosmétiquement acceptable et un dérivé de méthylène furanone selon la revendication 3 en tant qu'agent dépigmentant.

**16.** Composition dermocosmétique selon l'une des revendications 14 et 15 **caractérisée en ce que** la composition se présente sous forme de crème, gel ou spray.

**17.** Procédé de préparation de dérivés de méthylène furanone de formule générale (I) selon l'une quelconque des revendications 1 à 4
**caractérisé en ce qu'**il implique une réaction de couplage de Suzuki-Myaura à partir de dérivés dibromofuranones de formule générale (II) :

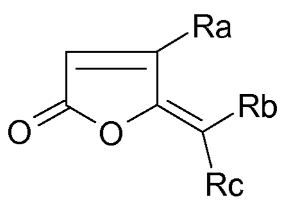

sous leurs formes isomères (E)ou (Z), pures ou en mélange,
dans laquelle
Ra représente un hydrogène et Rb, Rc représente un radical bromo.

**18.** Procédé de préparation des dérives de méthylène furanone de formule générale (I) tels que définis selon l'une des revendications 1 à 3 **caractérisés en ce qu'**il implique une réaction de couplage du 2-methoxy-furane avec une cétone suivie d'une déshydratation in situ ou avec un agent de déshydratation.

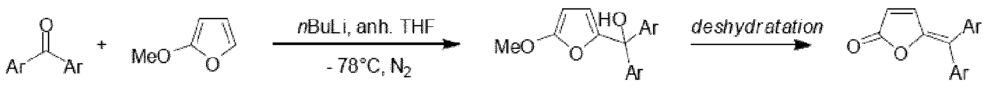


**Patentansprüche**

**1.** Methylenfuranon-Derivate der allgemeinen Formel (I):

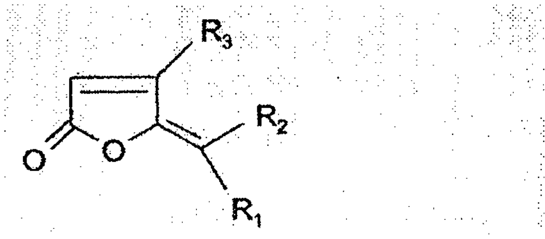

(I)

in Form ihrer (E)- oder (Z)-Isomeren, rein oder in Mischung, wobei

- $R_3$ einen Wasserstoff darstellt,
- $R_1$ und $R_2$ identisch sind und

    - einen Phenylrest, substituiert mit einem oder mehreren

        ◦ $(C_{1-5})$Alkyl-, $(C_{1-4})$Alkyloxy-,
        ◦ Hydroxy-,
        ◦ Methylthio-,
        ◦ Halogen-Rest(en) oder
        ◦ einer Aminogruppe, die gegebenenfalls mit einem $(C_{1-4})$Alkyl-, $(C_{1-4})$Alkyloxycarbonyl-Rest substituiert ist,

    - einen Thiophenylrest,
    - einen Thionaphthenylrest,
    - einen Thiazolylrest,
    - einen Pyridinylrest,
    - einen Benzomorpholinylrest,
    - einen Naphthylrest

darstellen,
jedoch mit der Maßgabe, dass $R_1$ und $R_2$ nicht gleichzeitig einen Paramethoxyphenylrest darstellen.

**2.** Methylenfuranon-Derivate der allgemeinen Formel (I) gemäß Anspruch 1, **dadurch gekennzeichnet, dass**

- $R_3$ einen Wasserstoff darstellt,
- $R_1$ und $R_2$ identisch sind und

    - einen Phenylrest, substituiert mit einem oder mehreren

- ($C_{1-4}$)Alkyloxy-,
- Hydroxy-Rest(en),

- einen Thiophenylrest,

darstellen,
jedoch mit der Maßgabe, dass $R_1$ und $R_2$ nicht gleichzeitig einen Paramethoxyphenylrest darstellen.

**3.** Methylenfuranon-Derivate der allgemeinen Formel (I) gemäß einem der Ansprüche 1 bis 2, **dadurch gekennzeichnet, dass**:

- $R_3$ einen Wasserstoff darstellt,
- $R_1$ und $R_2$ identisch sind und

  - einen Phenylrest, substituiert mit einem oder mehreren

    - Hydroxy-Rest(en),

darstellen.

**4.** Methylenfuranon-Derivate der allgemeinen Formel (I) gemäß einem der Ansprüche 1 bis 3, in Form ihrer (E)- oder (Z)-Isomeren, rein oder in Mischung, ausgewählt aus:

- 5-(Bis(3,4-dimethoxyphenyl)methylen)furan-2(5*H*)-on;
- 5-(Dithiophen-3-ylmethylen)furan-2(5*H*)-on;
- 5-(Bis(*tert*-butyl(4-hydroxyphenyl)carbamat)furan-2(5*H*)-on;
- 5-(Bis(2-fluorphenyl)methylen)furan-2(5*H*)-on;
- 5-(Bis(4-methylthio)phenyl)methylen)furan-2(5*H*)-on;
- 5-(Bis(4-dimethylamino)phenyl)methylen)furan-2(5*H*)-on;
- 5-(Bis(4-methyl-3,4-dihydro-2H-benzo[b][1,4]oxazin-7-yl)methylen)furan-2(5*H*)-on;
- 5-(Dithiazol-2-ylmethylen)furan-2(5*H*)-on;
- 5-(Bis(4-fluorphenyl)methylen)furan-2(5*H*)-on;
- 5-(Bis(4-hydroxy-3,5-dimethylphenyl)methylen)furan-2(5*H*)-on;
- 5-(Bis(3,4,5-trihydroxyphenyl)methylen)furan-2(5*H*)-on;
- 5-(Bis(3,4-dihydroxyphenyl)methylen)furan-2(5*H*)-on;
- 5-(Bis(4-hydroxy-3-methoxyphenyl)methylen)furan-2(5*H*)-on;
- 5-(Bis(4-hydroxy-3,5-dimethoxyphenyl)methylen)furan-2(5*H*)-on;
- 5-(Bis(4-hydroxyphenyl)methy(en)furan-2(5*H*)-on;
- 5-(Bis(2,4-dimethoxyphenyl)methylen)furan-2(5*H*)-on;
- 5-(Bis(2,4-dihydroxyphenyl)methylen)furan-2(5*H*)-on.

**5.** Kosmetische Verwendung von Methylenfuranon-Derivaten der allgemeinen Formel (I):

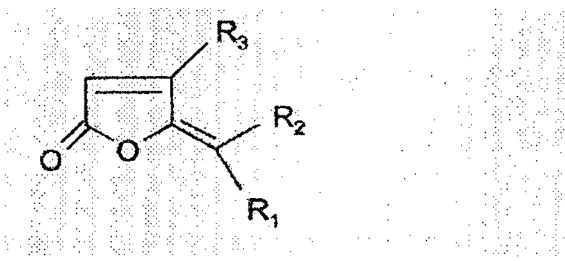

(I)

in Form ihrer (E)- oder (Z)-Isomeren, rein oder in Mischung, wobei einer der Reste $R_1$ und $R_3$ unabdingbar einen Wasserstoff darstellt, und $R_1$, $R_2$ und $R_3$ identisch oder unterschiedlich sind und

- einen Phenylrest, gegebenenfalls substituiert mit einem oder mehreren

◦ $(C_{1-5})$Alkyl-, $(C_{1-4})$Alkyloxy-,
◦ Hydroxy-,
◦ Methylthio-,
◦ Halogen-Rest(en) oder
◦ einer Aminogruppe, die gegebenenfalls mit einem $(C_{1-4})$Alkyl-, $(C_{1-4})$Alkyloxycarbonyl-Rest substituiert ist,

- einen Thiophenylrest,
- einen Thionaphthenylrest,
- einen Thiazolylrest,
- einen Pyridinylrest,
- einen Benzomorpholinylrest,
- einen Naphthylrest

darstellen,

- $R_1$ und $R_2$ miteinander verbunden sind und einen Fluorenylrest darstellen,

als Lichtschutzmittel.

**6.** Kosmetische Verwendung gemäß Anspruch 5, **dadurch gekennzeichnet, dass** die Methylenfuranon-Derivate der allgemeinen Formel (I) in Form ihrer (E)- oder (Z)-Isomeren, rein oder in Mischung, vorliegen, und **dadurch gekennzeichnet, dass**

- $R_3$ einen Wasserstoff darstellt, und
- $R_1$ und $R_2$ identisch oder unterschiedlich sind und

  - einen Phenylrest, gegebenenfalls substituiert mit einem oder mehreren

    ◦ $(C_{1-5})$Alkyl-, $(C_{1-4})$Alkyloxy-,
    ◦ Hydroxy-,
    ◦ Methylthio-,
    ◦ Halogen-Rest(en) oder
    ◦ einer Aminogruppe, die gegebenenfalls mit einem $(C_{1-4})$Alkyl-, $(C_{1-4})$Alkyloxycarbonyl-Rest substituiert ist,

  - einen Thiophenylrest,
  - einen Thionaphthenylrest,
  - einen Thiazolylrest,
  - einen Pyridinylrest,
  - einen Benzomorpholinylrest,
  - einen Naphthylrest

darstellen,

- $R_1$ und $R_2$ miteinander verbunden sind und einen Fluorenylrest darstellen.

**7.** Methylenfuranon-Derivate der allgemeinen Formel (I):

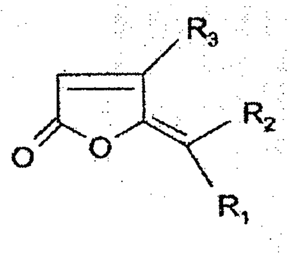

(I)

in Form ihrer (E)- oder (Z)-Isomeren, rein oder in Mischung, wobei einer der Reste $R_1$ und $R_3$ unabdingbar einen Wasserstoff darstellt, und $R_1$, $R_2$ und $R_3$ identisch oder unterschiedlich sind und

- einen Phenylrest, gegebenenfalls substituiert mit einem oder mehreren
    - ◦ $(C_{1-5})$Alkyl-, $(C_{1-4})$Alkyloxy-,
    - ◦ Hydroxy-,
    - ◦ Methylthio-,
    - ◦ Halogen-Rest(en) oder
    - ◦ einer Aminogruppe, die gegebenenfalls mit einem $(C_{1-4})$Alkyl-, $(C_{1-4})$Alkyloxycarbonyl-Rest substituiert ist,
- einen Thiophenylrest,
- einen Thionaphthenylrest,
- einen Thiazolylrest,
- einen Pyridinylrest,
- einen Benzomorpholinylrest,
- einen Naphthylrest

darstellen,

- $R_1$ und $R_2$ miteinander verbunden sind und einen Fluorenylrest darstellen,

zur dermatologischen Verwendung als Lichtschutzmittel.

**8.** Derivate zur Verwendung gemäß Anspruch 7, **dadurch gekennzeichnet, dass** die Methylenfuranon-Derivate der allgemeinen Formel (I) in Form ihrer (E)- oder (Z)-Isomeren, rein oder in Mischung, vorliegen, und **dadurch gekennzeichnet, dass**

- $R_3$ einen Wasserstoff darstellt, und
- $R_1$ und $R_2$ identisch oder unterschiedlich sind und
    - einen Phenylrest, gegebenenfalls substituiert mit einem oder mehreren
        - ◦ $(C_{1-5})$Alkyl-, $(C_{1-4})$Alkyloxy-,
        - ◦ Hydroxy-,
        - ◦ Methylthio-,
        - ◦ Halogen-Rest(en) oder
        - ◦ einer Aminogruppe, die gegebenenfalls mit einem $(C_{1-4})$Alkyl-, $(C_{1-4})$Alkyloxycarbonyl-Rest substituiert ist,
    - einen Thiophenylrest,
    - einen Thionaphthenylrest,
    - einen Thiazolylrest,
    - einen Pyridinylrest,
    - einen Benzomorpholinylrest,
    - einen Naphthylrest

darstellen,

- $R_1$ und $R_2$ miteinander verbunden sind und einen Fluorenylrest darstellen.

**9.** Kosmetische Verwendung von Methylenfuranon-Derivaten der allgemeinen Formel (I):

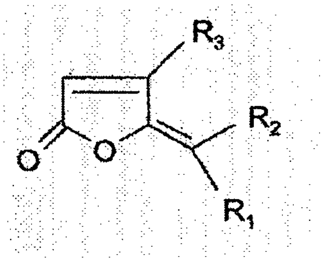

(I)

in Form ihrer (E)- oder (Z)-Isomeren, rein oder in Mischung, wobei einer der Reste $R_1$ und $R_3$ unabdingbar einen Wasserstoff darstellt,
und $R_1$, $R_2$ und $R_3$ identisch oder unterschiedlich sind und

- einen Phenylrest, gegebenenfalls substituiert mit einem oder mehreren
  - $(C_{1-5})$Alkyl-, $(C_{1-4})$Alkyloxy-,
  - Hydroxy-,
  - Methylthio-,
  - Halogen-Rest(en) oder
  - einer Aminogruppe, die gegebenenfalls mit einem $(C_{1-4})$Alkyl-, $(C_{1-4})$Alkyloxycarbonyl-Rest substituiert ist,
- einen Thiophenylrest,
- einen Thionaphthenylrest,
- einen Thiazolylrest,
- einen Pyridinylrest,
- einen Benzomorpholinylrest,
- einen Naphthylrest

darstellen,

- $R_1$ und $R_2$ miteinander verbunden sind und einen Fluorenylrest darstellen,

als Antioxidationsmittel.

**10.** Kosmetische Verwendung gemäß Anspruch 9, **dadurch gekennzeichnet, dass** die Methylenfuranon-Derivate der allgemeinen Formel (I) in Form ihrer (E)- oder (Z)-Isomeren, rein oder in Mischung, vorliegen, und **dadurch gekennzeichnet, dass**

- $R_3$ einen Wasserstoff darstellt, und
- $R_1$ und $R_2$ identisch oder unterschiedlich sind und
  - einen Phenylrest, gegebenenfalls substituiert mit einem oder mehreren
    - $(C_{1-5})$Alkyl-, $(C_{1-4})$Alkyloxy-,
    - Hydroxy-,
    - Methylthio-,
    - Halogen-Rest(en) oder
    - einer Aminogruppe, die gegebenenfalls mit einem $(C_{1-4})$Alkyl-, $(C_{1-4})$Alkyloxycarbonyl-Rest substituiert ist,
  - einen Thiophenylrest,
  - einen Thionaphthenylrest,
  - einen Thiazolylrest,
  - einen Pyridinylrest,
  - einen Benzomorpholinylrest,
  - einen Naphthylrest

darstellen,

- $R_1$ und $R_2$ miteinander verbunden sind und einen Fluorenylrest darstellen.

**11.** Methylenfuranon-Derivate der allgemeinen Formel (I):

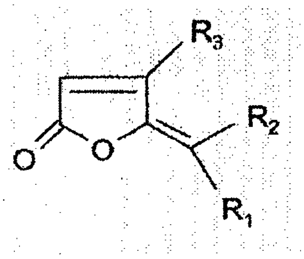

(I)

in Form ihrer (E)- oder (Z)-Isomeren, rein oder in Mischung, wobei einer der Reste $R_1$ und $R_3$ unabdingbar einen Wasserstoff darstellt, und $R_1$, $R_2$ und $R_3$ identisch oder unterschiedlich sind und

- einen Phenylrest, gegebenenfalls substituiert mit einem oder mehreren
  - ◦ ($C_{1-5}$)Alkyl-, ($C_{1-4}$)Alkyloxy-,
  - ◦ Hydroxy-,
  - ◦ Methylthio-,
  - ◦ Halogen-Rest(en) oder
  - ◦ einer Aminogruppe, die gegebenenfalls mit einem ($C_{1-4}$)Alkyl-, ($C_{1-4}$)Alkyloxycarbonyl-Rest substituiert ist,
- einen Thiophenylrest,
- einen Thionaphthenylrest,
- einen Thiazolylrest,
- einen Pyridinylrest,
- einen Benzomorpholinylrest,
- einen Naphthylrest

darstellen,

- $R_1$ und $R_2$ miteinander verbunden sind und einen Fluorenylrest darstellen,

zur dermatologischen Verwendung als Antioxidationsmittel.

**12.** Derivate zur Verwendung gemäß-Anspruch 11, **dadurch gekennzeichnet, dass** die Methylenfuranon-Derivate der allgemeinen Formel (I) in Form ihrer (E)- oder (Z)-Isomeren, rein oder in Mischung, vorliegen, und **dadurch gekennzeichnet, dass**

- $R_3$ einen Wasserstoff darstellt, und
- $R_1$ und $R_2$ identisch oder unterschiedlich sind und
  - einen Phenylrest, gegebenenfalls substituiert mit einem oder mehreren
    - ◦ ($C_{1-5}$)Alkyl-, ($C_{1-4}$)Alkyloxy-,
    - ◦ Hydroxy-,
    - ◦ Methylthio-,
    - ◦ Halogen-Rest(en) oder
    - ◦ einer Aminogruppe, die gegebenenfalls mit einem ($C_{1-4}$)Alkyl-, ($C_{1-4}$)Alkyloxycarbonyl-Rest substituiert ist,
  - einen Thiophenylrest,
  - einen Thionaphthenylrest,

- einen Thiazolylrest,
- einen Pyridinylrest,
- einen Benzomorpholinylrest,
- einen Naphthylrest

darstellen,

- $R_1$ und $R_2$ miteinander verbunden sind und einen Fluorenylrest darstellen.

**13.** Verwendung von Methylenfuranon-Derivaten der allgemeinen Formel (I) gemäß Anspruch 3 als Depigmentierungsmittel.

**14.** Dermokosmetische Zusammensetzung, welche einen kosmetisch akzeptablen Trägerstoff und ein Methylenfuranon-Derivat gemäß irgendeinem der Ansprüche 1 bis 4 als Lichtschutzmittel oder Antioxidationsmittel enthält.

**15.** Dermokosmetische Zusammensetzung, welche einen kosmetisch akzeptablen Trägerstoff und ein Methylenfuranon-Derivat gemäß Anspruch 3 als Depigmentierungsmittel enthält.

**16.** Dermokosmetische Zusammensetzung gemäß einem der Ansprüche 14 und 15, **dadurch gekennzeichnet, dass** die Zusammensetzung in Form einer Creme, eines Gels oder eines Sprays vorliegt.

**17.** Verfahren zur Herstellung von Methylenfuranon-Derivaten der allgemeinen Formel (I) gemäß irgendeinem der Ansprüche 1 bis 4, **dadurch gekennzeichnet, dass** es eine Suzuki-Myaura-Kopplungsreaktion ausgehend von Dibromfuranonen der allgemeinen Formel (II):

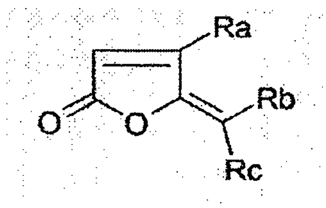

in Form ihrer (E)- oder (Z)-Isomeren, rein oder in Mischung, wobei
Ra einen Wasserstoff darstellt und Rb, Rc einen Bromrest darstellen, beinhaltet.

**18.** Verfahren zur Herstellung von Methylenfuranon-Derivaten der allgemeinen Formel (I), wie in einem der Ansprüche 1 bis 3 definiert, **dadurch gekennzeichnet, dass** es eine Kopplungsreaktion von 2-Methoxyfuran mit einem Keton gefolgt von einer Dehydratisierung *in situ* oder mit einem Dehydratisierungsmittel beinhaltet.

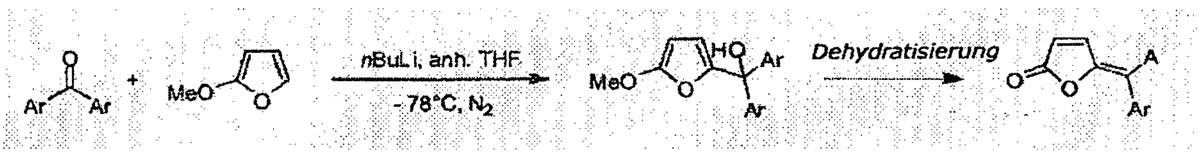


## Claims

**1.** Methylene furanone derivatives of general formula (I) :

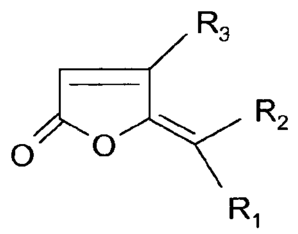

(I)

in their (E) or (Z) **isomer** forms, pure or in mixture, wherein

- R3 represents hydrogen
    - $R_1$ and $R_2$ are identical and represent
        - a phenyl radical substituted by one or more of the following radicals
            - ◦ $(C_{1-5})$ alkyl, $(c_{1-4})$ alkoxy,
            - ◦ hydroxy,
            - ◦ methylthio,
            - ◦ halogen, or
            - ◦ an amino group optionally substituted by a $(C_{1-4})$ alkyl or $(C_{1-4})$ alkoxycarbonyl radical,
        - a thiophenyl radical,
        - a thionaphthenyl radical,
        - a thiazolyl radical,
        - a pyridinyl radical,
        - a benzomorpholinyl radical,
        - a naphthyl radical

and
on the condition, however, that $R_1$ and $R_2$ do not simultaneously represent a paramethoxyphenyl radical.

**2.** The methylene furanone derivatives of general formula (I) according to claim 1 **characterized in that**:

- $R_3$ represents hydrogen
- $R_1$ and $R_2$ are identical and represent
    - a phenyl radical substituted by one or more of the following radicals
        - $(C_{1-4})$ alkoxy,
        - hydroxy,
    - a thiophenyl radical,

and on the condition, however, that $R_1$ and $R_2$ do not simultaneously represent a paramethoxyphenyl radical.

**3.** The methylene furanone derivatives of general formula (I) according to one of claims 1 to 2 **characterized in that**:

- $R_3$ represents hydrogen
- $R_1$ and $R_2$ are identical and represent
    - a phenyl radical substituted by one or more hydroxy radicals.

**4.** The methylene furanone derivatives of general formula (I) according to one of claims 1 to 3, in their (E) or (Z) isomer forms, pure or in mixture, selected among:

- 5-(bis(3,4-dimethoxyphenyl)methylene)furan-2(5*H*)-one;

- 5-(dithiophene-3-ylmethylene)furan-2(5*H*)-one;
- 5-bis(tert-butyl-4-hydroxyphenylcarbamate)furan-2 (5*H*) -one;
- 5-(bis(2-fluorophenyl)methylene)furan-2(5*H*)-one;
- 5-(bis(4-(methylthio)phenyl)methylene)furan-2(5*H*)-one;
- 5-(bis(4-(dimethylamino)phenyl)methylene)furan-2(5*H*)-one;
- 5-(bis(4-methyl-3,4-dihydro-2H-benzo[b][1,4]oxazin-7-yl)methylene)furan-2(5*H*)-one;
- 5-(dithiazol-2-ylmethylene)furan-2(5*H*)-one;
- 5-(bis (4-fluorophenyl)methylene) furan-2 (5*H*)-one;
- 5- (bis (4-hydroxy-3, 5-dimethylphenyl)methylene)furan-2(5*H*)-one;
- 5-(bis(3,4,5-trihydroxyphenyl)methylene)furan-2(5*H*)-one;
- 5-(bis(3,4-dihydroxyphenyl)methylene)furan-2(5*H*)-one;
- 5-(bis(4-hydroxy-3-methoxyphenyl)methylene)furan-2(5*H*)-one;
- 5-(bis(4-hydroxy-3,5-dimethoxyphenyl)methylene)furan-2(5*H*)-one;
- 5-(bis(4-hydroxyphenyl)methylene)furan-2(5*H*)-one;
- 5-(bis(2,4-dimethoxyphenyl)methylene)furan-2(5*H*)-one;
- 5-(bis(2,4-dihydroxyphenyl)methylene)furan-2(5*H*)-one.

**5.** A cosmetic use of methylene furanone derivatives of general formula (I):

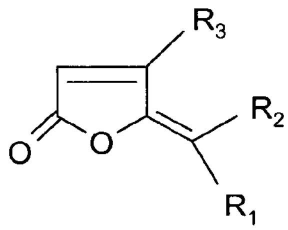

(I)

in their (E) or (Z) isomer forms, pure or in mixture, wherein one of the radicals $R_1$ and $R_3$ necessarily represents hydrogen, and $R_1$, $R_2$, $R_3$ are identical or different and represent:

- a phenyl radical optionally substituted by one or more of the following radicals
  - ◦ ($C_{1-5}$) alkyl, ($C_{1-4}$) alkoxy,
  - ◦ hydroxy,
  - ◦ methylthio,
  - ◦ halogen, or
  - ◦ an amino group optionally substituted by a ($C_{1-4}$) alkyl or ($C_{1-4}$) alkoxycarbonyl radical,
- a thiophenyl radical,
- a thionaphthenyl radical,
- a thiazolyl radical,
- a pyridinyl radical,
- a benzomorpholinyl radical,
- a naphthyl radical.
- $R_1$ and $R_2$ are bonded together and represent a fluorenyl radical

as a photoprotecting agent.

**6.** The cosmetic use according to claim 5, **characterized in that** the methylene furanone derivatives of general formula (I) are in their (E) or (Z) isomer forms, pure or in mixture, and **characterized in that**

- $R_3$ represents hydrogen, and
- $R_1$ and $R_2$ are identical or different and represent
  - a phenyl radical optionally substituted by one or more of the following radicals

◦ ($C_{1-5}$) alkyl, ($C_{1-4}$) alkoxy,
◦ hydroxy,
◦ methylthio,
◦ halogen, or
◦ an amino group optionally substituted by a ($C_{1-4}$) alkyl or ($C_{1-4}$) alkoxycarbonyl radical,

- a thiophenyl radical,
- a thionaphthenyl radical,
- a thiazolyl radical,
- a pyridinyl radical,
- a benzomorpholinyl radical,
- a naphthyl radical.
- $R_1$ and $R_2$ are bonded together and represent a fluorenyl radical.

**7.** The methylene furanone derivatives of general formula (I) :

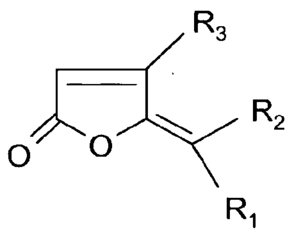

(I)

in their (E) or (Z) isomer forms, pure or in mixture, wherein one of the radicals $R_1$ and $R_3$ necessarily represents hydrogen, and $R_1$, $R_2$, $R_3$ are identical or different and represent:

- a phenyl radical optionally substituted by one or more of the following radicals

◦ ($C_{1-5}$) alkyl, ($C_{1-4}$) alkoxy,
◦ hydroxy,
◦ methylthio,
◦ halogen, or
◦ an amino group optionally substituted by a ($C_{1-4}$) alkyl or ($C_{1-4}$) alkoxycarbonyl radical,

- a thiophenyl radical,
- a thionaphthenyl radical,
- a thiazolyl radical,
- a pyridinyl radical,
- a benzomorpholinyl radical,
- a naphthyl radical.
- $R_1$ and $R_2$ are bonded together and represent a fluorenyl radical

for dermatological use as a photoprotecting agent.

**8.** The derivatives for use according to claim 7, **characterized in that** the methylene furanone derivatives of general formula (I) are in their (E) or (Z) isomer forms, pure or in mixture, and **characterized in that**

- $R_3$ represents hydrogen, and
- $R_1$ and $R_2$ are identical or different and represent

- a phenyl radical optionally substituted by one or more of the following radicals

◦ ($C_{1-5}$) alkyl, ($C_{1-4}$) alkoxy,
◦ hydroxy,

- ◦ methylthio,
- ◦ halogen, or
- ◦ an amino group optionally substituted by a ($C_{1-4}$) alkyl or ($C_{1-4}$) alkoxycarbonyl radical,

- a thiophenyl radical,
- a thionaphthenyl radical,
- a thiazolyl radical,
- a pyridinyl radical,
- a benzomorpholinyl radical,
- a naphthyl radical.
- $R_1$ and $R_2$ are bonded together and represent a fluorenyl radical.

**9.** A cosmetic use of methylene furanone derivatives of general formula (I):

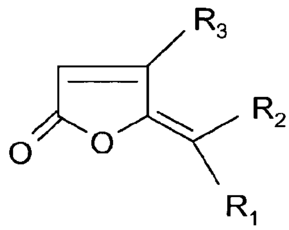

(I)

in their (E) or (Z) isomer forms, pure or in mixture, wherein
one of the radicals $R_1$ and $R_3$ necessarily represents hydrogen,
and $R_1$, $R_2$, $R_3$ are identical or different and represent:

- a phenyl radical optionally substituted by one or more of the following radicals

  - ◦ ($C_{1-5}$) alkyl, ($C_{1-4}$) alkoxy,
  - ◦ hydroxy,
  - ◦ methylthio,
  - ◦ halogen, or
  - ◦ an amino group optionally substituted by a ($C_{1-4}$) alkyl or ($C_{1-4}$) alkoxycarbonyl radical,

- a thiophenyl radical,
- a thionaphthenyl radical,
- a thiazolyl radical,
- a pyridinyl radical,
- a benzomorpholinyl radical,
- a naphthyl radical.
- $R_1$ and $R_2$ are bonded together and represent a fluorenyl radical

as an antioxidant agent.

**10.** The cosmetic use according to claim 9, **characterized in that** the methylene furanone derivatives of general formula (I) are in their (E) or (Z) isomer forms, pure or in mixture,
and **characterized in that**

- $R_3$ represents hydrogen, and
- $R_1$ and $R_2$ are identical or different and represent
- a phenyl radical optionally substituted by one or more of the following radicals

  - ◦ ($C_{1-5}$) alkyl, ($C_{1-4}$) alkoxy,
  - ◦ hydroxy,
  - ◦ methylthio,
  - ◦ halogen, or
  - ◦ an amino group optionally substituted by a ($C_{1-4}$) alkyl or ($C_{1-4}$) alkoxycarbonyl radical,

- a thiophenyl radical,
- a thionaphthenyl radical,
- a thiazolyl radical,
- a pyridinyl radical,
- a benzomorpholinyl radical,
- a naphthyl radical.
- $R_1$ and $R_2$ are bonded together and represent a fluorenyl radical.

**11.** The methylene furanone derivatives of general formula (I):

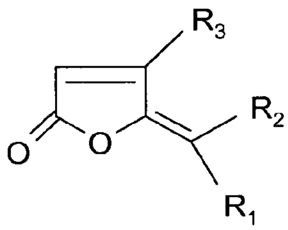

(I)

in their (E) or (Z) isomer forms, pure or in mixture, wherein one of the radicals $R_1$ and $R_3$ necessarily represents hydrogen, and $R_1$, $R_2$, $R_3$ are identical or different and represent:

- a phenyl radical optionally substituted by one or more of the following radicals
    - ◦ ($C_{1-5}$) alkyl, ($C_{1-4}$) alkoxy,
    - ◦ hydroxy,
    - ◦ methylthio,
    - ◦ halogen, or
    - ◦ an amino group optionally substituted by a ($C_{1-4}$) alkyl or ($C_{1-4}$) alkoxycarbonyl radical,
- a thiophenyl radical,
- a thionaphthenyl radical,
- a thiazolyl radical,
- a pyridinyl radical,
- a benzomorpholinyl radical,
- a naphthyl radical.
- $R_1$ and $R_2$ are bonded together and represent a fluorenyl radical

for dermatological use as an antioxidant agent.

**12.** The derivatives for use according to claim 11, **characterized in that** the methylene furanone derivatives of general formula (I) are in their (E) or (Z) isomer forms, pure or in mixture, and **characterized in that**

- $R_3$ represents hydrogen, and
- $R_1$ and $R_2$ are identical or different and represent
    - a phenyl radical optionally substituted by one or more of the following radicals
        - ◦ ($C_{1-5}$) alkyl, ($C_{1-4}$) alkoxy,
        - ◦ hydroxy,
        - ◦ methylthio,
        - ◦ halogen, or
        - ◦ an amino group optionally substituted by a ($C_{1-4}$) alkyl or ($C_{1-4}$) alkoxycarbonyl radical,
    - a thiophenyl radical,
    - a thionaphthenyl radical,

- a thiazolyl radical,
- a pyridinyl radical,
- a benzomorpholinyl radical,
- a naphthyl radical.

$R_1$ and $R_2$ are bonded together and represent a fluorenyl radical.

**13.** Use of methylene furanone derivatives of general formula (I) according to claim 3 as a depigmentation agent.

**14.** A dermocosmetic composition comprising a cosmetically acceptable excipient and a methylene furanone derivative according to any one of claim 1 to 4 as a photoprotecting agent or an antioxidant agent.

**15.** A dermocosmetic composition comprising a cosmetically acceptable excipient and a methylene furanone derivative according to claim 3 as a depigmentation agent.

**16.** The dermocosmetic composition according to one of claims 14 and 15 **characterized in that** the composition is provided in cream, gel or spray form.

**17.** A method for preparing methylene furanone derivatives of general formula (I) according to any one of claims 1 to 4 **characterized in that** it involves a Suzuki-Miyaura coupling reaction from dibromofuranone derivatives of general formula (II):

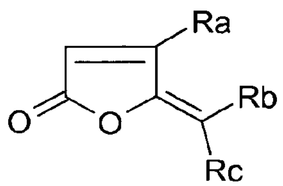

in their (E) or (Z) isomer forms, pure or in mixture, wherein
Ra represents hydrogen and Rb and Rc represent a bromo radical.

**18.** A method for preparing methylene furanone derivatives of general formula (I) such as defined according to one of claims 1 to 3 **characterized in that** it involves a coupling reaction of 2-methoxy-furan with a ketone followed by dehydration *in situ* or with a dehydration agent.

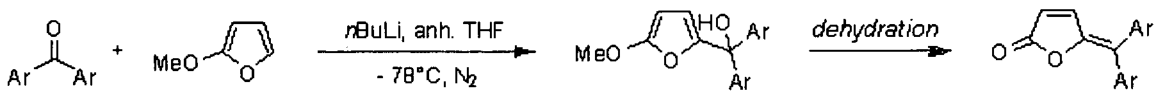

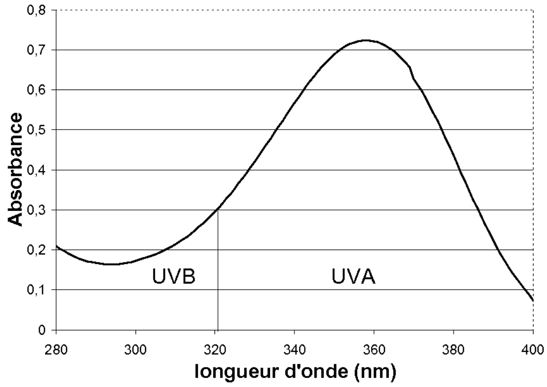


Figure 1

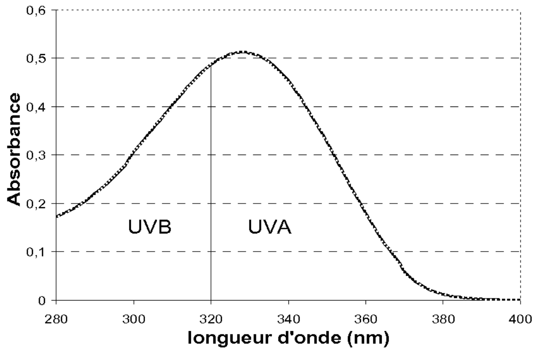


Figure 2

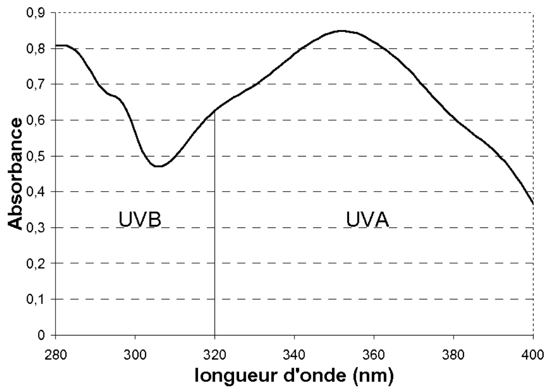


Figure 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6258963 B1


### Littérature non-brevet citée dans la description


- **MANNY, A. J. ; KJELLEBERG, S. ; KUMAR, N. ; DE NYS, R. ; READ, R. W. ; STEINBERG, P.** *Tetrahedron,* 1997, vol. 53, 158813-15826
- **SORG, A. ; SIEGEL, K. ; BRÜCKNER, R.** *Synlett,* 2004, vol. 2, 231-325
- *JACS,* 1986, 7055
- *Synth. Comm.,* 2008, 212
- *Eur. J. Org. Chem.,* 2004, 2381
- *Tet.,* 2005, 2055
- *Synthesis,* 2000, 1253